(19) Europäisches Patentamt
European Patent Office
Office européen des brevets

(11) EP 3 970 796 B1

(12) **EUROPEAN PATENT SPECIFICATION**

(45) Date of publication and mention of the grant of the patent:
**20.08.2025 Bulletin 2025/34**

(21) Application number: **20806275.2**

(22) Date of filing: **14.05.2020**

(51) International Patent Classification (IPC):
***A61K 38/22*** *(2006.01)* ***A61K 38/16*** *(2006.01)*
***C07K 14/47*** *(2006.01)* ***C12N 5/0793*** *(2010.01)*
***C07K 14/575*** *(2006.01)* ***A61P 27/02*** *(2006.01)*
***A61P 29/00*** *(2006.01)*

(52) Cooperative Patent Classification (CPC):
**A61P 27/02; A61K 38/2278; A61P 29/00; C07K 14/57563; C12N 5/0619**

(86) International application number:
**PCT/JP2020/019347**

(87) International publication number:
**WO 2020/230869 (19.11.2020 Gazette 2020/47)**

(54) **COMPOSITION FOR PREVENTING OR TREATING NEUROTROPHIC KERATITIS WHICH CONTAINS STABILIZED PACAP PEPTIDE**

ZUSAMMENSETZUNG ZUR VORBEUGUNG ODER BEHANDLUNG NEUROTROPHER KERATITIS MIT EINEM STABILISIERTEM PACAP-PEPTID

COMPOSITION POUR PRÉVENIR OU TRAITER UNE KÉRATITE NEUROTROPHIQUE CONTENANT UN PEPTIDE DE TYPE PACAP STABILISÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.05.2019 JP 2019091700**

(43) Date of publication of application:
**23.03.2022 Bulletin 2022/12**

(73) Proprietor: **Senju Pharmaceutical Co., Ltd.**
**Osaka-shi**
**Osaka 541-0048 (JP)**

(72) Inventors:
- **NAKAJIMA, Takeshi**
  **Osaka-shi, Osaka 541-0048 (JP)**
- **MACHIDA, Mamiko**
  **Osaka-shi, Osaka 541-0048 (JP)**
- **YAMADA, Shuhei**
  **Osaka-shi, Osaka 541-0048 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 749 291** **WO-A1-2005/102375**
**WO-A1-2019/046876** **US-A1- 2006 270 592**

- **MANTUREWICZ M ET AL: "Tetrazole Analogues of Aspartic, Glutamic and .alpha.-Aminoadipic Acids and Their Derivatives with Tetrazole Ring in Side Chains Useful for Solid-Phase Peptide Synthesis", POLISH JOURNAL OF CHEMISTRY, POLSKIE TOWARZYSTWO CHEMICZNE, POLISH CHEMICAL SOCIETY, PL, vol. 81, no. 12, 1 January 2007 (2007-01-01), pages 2121 - 2131, XP008121890, ISSN: 0137-5083**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **FUKIAGE ET AL: "PACAP Induces Neurite Outgrowth in Cultured Trigeminal Ganglion Cells and Recovery of Corneal Sensitivity After Flap Surgery in Rabbits", AMERICAN JOURNAL OF OPHTHALMOLOGY, ELSEVIER, AMSTERDAM, NL, vol. 143, no. 2, 25 January 2007 (2007-01-25), pages 255 - 262.e1, XP005862662, ISSN: 0002-9394, DOI: 10.1016/J.AJO.2006.10.034**
- **LEONARDO MASTROPASQUA, MASSARO-GIORDANO GIACOMINA, NUBILE MARIO, SACCHETTI MARTA: "Understanding the pathogenesis of neurotrophic keratitis: The role of corneal nerves", JOURNAL OF CELLULAR PHYSIOLOGY, vol. 232, no. 4, 1 April 2017 (2017-04-01), pages 717 - 724, XP055760443, ISSN: 0021-9541, DOI: 10.1002/jcp.25623**
- **MCILVAIN H B; BAUDY A; SULLIVAN K; LIU D; PONG K; FENNELL M; DUNLOP J: "Pituitary adenylate cyclase-activating peptide (PACAP) induces differentiation in the neuronal FII cell line through a PKA-dependent pathway", BRAIN RESEARCH, vol. 1077, no. 1, 10 March 2006 (2006-03-10), pages 16 - 23, XP027917435, ISSN: 0006-8993, DOI: 10.1016/j.brainres.2005.12.130**
- **SATOMI ONOUE, WAKI YOSHIHIRO, NAGANO YUMIKO, SATOH SEIJI, KASHIMOTO KAZUHISA: "The neuromodulatory effects of VIP/PACAP on PC-12 cells are associated with their N-terminal structures", PEPTIDES, vol. 22, no. 6, 2001, pages 867 - 872, XP055760449, ISSN: 0196-9781, DOI: 10.1016/S0196-9781(01)00411-9**


Remarks:

The complete document including Reference Table(s) and the Sequence Listing(s) can be downloaded from the EPO website

## Description

TECHNICAL FIELD

The present invention relates to a peptide as claimed for preventing or treating neurotrophic keratitis comprising a stabilized peptide with PACAP physiological activity.

BACKGROUND ART

Corneal sensation is important for providing damage prevention by the reflexes of blinking and tear secretion, and this sensation is governed by the corneal nerve. The corneal nerve not only mediates sensation but also affects corneal epithelial cell growth via secretion of neurotransmitters released from corneal nerve endings in the corneal epithelium, and nerve growth factors. When the corneal nerve is damaged, corneal epithelial cell metabolism is altered and proliferative ability is lost, and this is known to cause trophic ulcers. Neurotrophic keratitis and neuroparalytic keratopathy are degenerative corneal diseases resulting from damage to the trigeminal nerve, and they lead to damage of the corneal epithelium. The most severe type may lead to corneal ulceration, fusion or perforation, and loss of vision by the patient. Neurotrophic keratitis can result from a variety of clinical symptoms including viral infection such as corneal herpes, eye injury, corneal surgery or systemic conditions such as diabetes. Herpes infection, in particular, has been reported to be responsible for 27% of neurotrophic keratitis cases. Neurotrophic keratitis has long been recognized but no effective treatment method has yet been developed.

Advances in neuroscience have led to the discovery of numerous neuroprotective factors, and they are expected for developing as a preventive or therapeutic agent for nervous disorder. It has been discovered that drugs that reduce free radicals and excitatory amino acids, which are causes of neurodegeneration, or that are able to protect and/or repair neurons (for example, immunophilin ligands such as neurotrophic factors or immunosuppressive agents) have neuroprotective action, and *in vivo* proteins such as pituitary adenylate cyclase-activating polypeptide (PACAP), CD44 and human brain carboxypeptidase B (HBCPB) have been shown to exhibit neuroprotective action (PTLs 1 and 2).

Pituitary adenylate cyclase-activating polypeptide (PACAP) is a neuropeptide originally discovered in sheep hypothalamus extract. PACAP can exhibit activity which stimulates cAMP formation in anterior pituitary cells. PACAP includes PACAP38 which consists of 38 amino acid residues, and PACAP27 which consists of 27 amino acid residues, both of which have equivalent activity (NPLs 1 and 2). PACAP belongs to the vasoactive intestinal polypeptide (VIP)/secretin/glucagon superfamily, and the sequence of human PACAP27 has 68% identity with vasoactive intestinal polypeptide (VIP). PACAP and VIP both bind to PAC1 receptor (PAC1R), VPAC1 receptor (VPAC1R) and VPAC2 receptor (VPAC2R), but they differ in their affinities for their respective receptors. PAC1R binds to PACAP with high selectivity, with an affinity for PACAP that is more than 1000 times higher than its affinity for VIP. VPAC1R and VPAC2R, on the other hand, both have equal affinity for PACAP and VIP. PACAP has a variety of physiological effects, being known to have physiological activity as a neuroprotective substance, immunosuppressive factor, vasodilating factor, exocrine secretagogue (PTL 3) and neurite formation promoting factor (PTL 4).

Medical drug is being developed utilizing the various physiological activities of PACAP. However, relatively short peptides such as PACAP are unstable in aqueous solution and are associated with the problem of a short half-life due to lack of protease resistance.

[CITATION LIST]

[PATENT LITERATURE]

[PTL 1] Japanese Patent Public Inspection No. 2014-510101
[PTL 2] Japanese Unexamined Patent Publication No. 2012-232952
[PTL 3] Japanese Unexamined Patent Publication No. 2009-269818
[PTL 4] International Patent Publication No. 2005/102375

[NON PATENT LITERATURE]

[NPL 1] S. Bourgault (2009) Current Medicinal Chemistry 16, 4462-4480
[NPL 2] Louise Dickson (2009) Pharmacology & Therapeutics, 12, 294-316

[NPL 3] Alessandro Lambiase (2012) Investigative Ophthalmology & Visual Science, vol. 53, No. 13, p.8280-8287

SUMMARY OF INVENTION

[TECHNICAL PROBLEM]

It is an object of the present invention to provide a peptide as claimed for preventing or treating neurotrophic keratitis.

[SOLUTION TO PROBLEM]

The present inventors have invented this invention upon finding that PACAP peptide or its stabilized peptide exhibits an inhibiting effect against corneal damage in a neurotrophic keratitis model, and promotes nerve axon elongation in a cultured nerve cell model. It was further found that stability in aqueous solutions can be drastically increased by replacing the carboxyl groups of aspartic acid in the PACAP peptide with tetrazole, making it a stabilized peptide.
The present invention is set out in the claims.

[ADVANTAGEOUS EFFECTS OF INVENTION]

According to the invention, stabilized PACAP can be used to prevent or treat neurotrophic keratitis, or to promote nerve axon elongation.

BRIEF DESCRIPTION OF DRAWINGS

Fig. 1 shows the cAMP inducing effects of different peptides (peptides 1, 2 and 6 to 9) in a PAC1R high-expressing cell line.
Fig. 2 shows the cAMP inducing effects of different peptides (peptides 1, 2 and 6 to 9) in a VPAC1R high-expressing cell line.
Fig. 3 shows the lacrimal secretion-promoting effects of different peptides (peptide 1 and peptide 9) in a neurotrophic keratitis rat model.
Fig. 4 shows changes in superficial punctate keratitis (SPK) scores by different peptides (peptide 1 and peptide 9) in a neurotrophic keratitis rat model.
Fig. 5 shows axon elongation of cultured trigeminal nerves by different peptides.

DESCRIPTION OF EMBODIMENTS

The present invention relates to a stabilized PACAP peptide as claimed for treatment or prevention of neurotrophic keratitis. According to another aspect, the invention relates to a method of promoting nerve axon elongation *in vitro* as claimed, comprising application of a stabilized PACAP peptide. A stabilized PACAP peptide is a peptide that has modification of amino acids to increase stability, while maintaining PACAP physiological activity. Specifically, the stabilized PACAP peptide consisting of the amino acid sequence of PACAP peptide wherein the carboxyl groups of the aspartic acid residues at position 3 and/or position 8 have been replaced by tetrazole, or its partially modified sequence. The stabilized PACAP peptide of the invention has affinity for PAC1R, VPAC1R and/or VPAC2R that is equivalent to that of PACAP. Equivalent affinity means that the EC50 values of the peptide for each of the receptors is within 10 times, preferably within 5 times and more preferably within 3 times, that of PACAP. The PACAP may be PACAP38 consisting of 38 residues, or PACAP27 consisting of 27 residues. PACAP38 and PACAP27 have the following sequences:

[Chemical Formula 1] **PACAP38 : HSDGIFTDSYSRYRKQMAVKKYLAAVLGKRYKQRVKNK (SEQ ID NO: 1) PACAP27 : HSDGIFTDSYSRYRKQMAVKKYLAAVL (SEQ ID NO: 2)**

According to the invention, a "sequence which is partially modified" is a sequence in which one or more amino acids of the original sequence have been substituted, deleted or added. More preferably, a "sequence which is partially modified" is a sequence in which one or several amino acids of the original sequence have been substituted, deleted or added.
An amino acid substitution may be at any position so long as the affinity of the peptide consisting of the sequence which is partially modified, for PAC1R, VPAC1R and/or VPAC2R, does not change. From the viewpoint of maintaining

affinity, the amino acid substitution in the peptide consisting of the sequence which is partially modified may be an amino acid substitution at any position among position 2, position 9, position 11, position 15 to position 17, position 19 to position 21 and position 27 of PACAP27. According to a particularly preferred aspect, the amino acids at positions $X_1$ to $X_{10}$ in the following sequence:
H-$X_1$-D-G-I-F-T-D-$X_2$-Y-$X_3$-R-Y-R-$X_4$-$X_5$-$X_6$-A-$X_7$-$X_8$-$X_9$-Y-L-A-A-V-$X_{10}$ (SEQ ID NO: 3) may be substituted.

The amino acid substitution may be a substitution of one or more amino acids, but from the viewpoint of activity, any number from 1 to 10, such as 1, 2, 3, 4, 5, 6, 7, 8, 9 or 10, amino acids may be substituted. A substitution of 1 to 4 amino acids is particularly preferred, a substitution of 3 amino acids is more preferred, a substitution of two amino acids is even more preferred and a substitution of one amino acid is most preferred.

An amino acid deletion may be at any position so long as the affinity of the peptide consisting of the sequence which is partially modified, for PAC1R, VPAC1R and/or VPAC2R, does not change. The number of amino acids deleted is selected as any number from 1 to 10, such as 1, 2, 3, 4, 5, 6, 7, 8, 9 or 10. A deletion of one or two amino acids is particularly preferred from the viewpoint of not altering affinity. The amino acid deletion may be a deletion at the N-terminal end or C-terminal end of the original sequence, or a deletion within the sequence. Since PACAP27 and PACAP38 have equivalent binding affinity for PAC1R, VPAC1R and/or VPAC2R, it is believed that deletion of amino acids present at the C-terminal end of PACAP38 have little effect on the affinity.

An amino acid addition may be at any position so long as the affinity of the peptide consisting of the sequence which is partially modified, for PAC1R, VPAC1R and/or VPAC2R, does not change. The number of amino acids added is selected as any number from 1 to 10, such as 1, 2, 3, 4, 5, 6, 7, 8, 9 or 10. The amino acids may be added at the N-terminal end or C-terminal end of the original sequence, or they may be added within the sequence. Since PACAP27 and PACAP38 have equivalent binding affinity for PAC1R, VPAC1R and/or VPAC2R, it is believed that addition of any amino acids at the C-terminus of PACAP27 has little effect on the affinity.

One aspect of the invention relates to a peptide as claimed consisting of the sequence represented by:H-X1-D-G-I-F-T-D-X2-Y-X3-R-Y-R-X4-X5-X6-A-X7-X8-X9-Y-L-A-A-V-X10 (SEQ **ID** NO: 3){where $X_1$ is a neutral amino acid, $X_2$ is a neutral amino acid, $X_3$ is a neutral amino acid, $X_4$ is a basic amino acid, $X_5$ is a neutral amino acid or egTz, $X_6$ is a non-polar amino acid, $X_7$ is a non-polar amino acid, $X_8$ is a basic amino acid, $X_9$ is a basic amino acid and $X_{10}$is a non-polar amino acid},with the carboxyl groups of the aspartic acid residues at position 3 and/or position 8 replaced by tetrazole, or its modified sequence, and having affinity for PAC1R, VPAC1R and/or VPAC2R.

The amino acids represented by $X_1$ to $X_{10}$ may be conservatively substituted by amino acids with the same attributes. As examples, conservative substitutions are substitutions of amino acids within the following groups:

1. non-polar amino acids Val, Leu, Ile, Met, Phe, Trp, Pro, Nle, Ala
2. neutral amino acids: Ala, Ser, Thr, Tyr, Cys, Asn, Gln, Gly
3. basic amino acids: Lys, Arg, His
4. acidic amino acids: Asp, Glu

Neutral amino acids for $X_1$, $X_2$ and $X_3$ are Ala, Ser, Thr, Tyr, Cys, Asn, Gln or Gly, and more preferably Ala or Ser.

Basic amino acids for $X_4$, $X_8$ and $X_9$ are Lys, Arg or His, and more preferably Lys or Arg.

A neutral amino acid for $X_5$ is Ala, Ser, Thr, Tyr, Cys, Asn, Gln or Gly, and more preferably Ala or Gln. $X_5$ may also be an amino acid having the amide of glutamine replaced with tetrazole (egTz).

A non-polar amino acid for $X_6$ is Val, Leu, Ile, Met, Phe, Trp, Pro, Nle or Ala, and more preferably Met, Nle, Leu or Ala.

A non-polar amino acid for $X_7$ is Val, Leu, Ile, Met, Phe, Trp, Pro, Nle or Ala, and more preferably Val or Ala.

A non-polar amino acid for $X_{10}$ is Val, Leu, Ile, Met, Phe, Trp, Pro, Nle or Ala, and more preferably Leu or Ala.

According to the invention, a "modified sequence" is a sequence in which one or more amino acids of the original sequence have been substituted, deleted or added. More preferably, a "modified sequence" is a sequence in which one or several amino acids of the original sequence have been substituted, deleted or added.

A modified sequence of the sequence listed as SEQ ID NO: 3 wherein the carboxyl groups of the aspartic acid residues at position 3 and/or position 8 have been replaced with tetrazole, is preferably the amino acid sequence listed as SEQ ID NO: 3 with a deletion or addition of one or more amino acids. More preferably, one or two amino acids may be deleted from the amino acid sequence listed as SEQ ID NO: 3, or the following C-terminal added sequence may be added to the amino acid sequence listed as SEQ ID NO: 3.

While it is not our intention to be limited to any particular theory, but since PACAP27 and PACAP38 have equivalent binding affinity for PAC1R, VPAC1R and/or VPAC2R, addition of any amino acids at the C-terminal end of PACAP27 does not effect the binding affinity for PAC1R. The PACAP27 stabilized of the invention may therefore further include a C-terminal added sequence, or it may lack a C-terminal added sequence. A C-terminal added sequence is a sequence comprising 1 to 11 arbitrary amino acids. The C-terminal added sequence preferably corresponds to the amino acids at position 28 to position 38 of PACAP38. The following sequences may therefore be mentioned as C-terminal

sequences:

GKRYKQRVKNK (SEQ ID NO: 37);
GKRYKQRVKN (SEQ ID NO: 38);
GKRYKQRVK (SEQ ID NO: 39);
GKRYKQRV (SEQ ID NO: 40);
GKRYKQR (SEQ ID NO: 41);
GKRYKQ (SEQ ID NO: 42);
GKRYK (SEQ ID NO: 43);
GKRY (SEQ ID NO: 44)
GKR;
GRR;
GK;
GR; and
G.

The peptide of the invention may use either the D-form or L-form of amino acids, or the racemic forms of amino acids, so long as affinity for PAC1R, VPAC1R and/or VPAC2R is not lost. The peptide of the invention may also comprise non-natural amino acids such as 2-aminoisobutyric acid or L-2-aminobutyric acid, and may include derivatives with optional modification of N-terminal amino groups, C-terminal carboxyl groups or amino acid side chain functional groups. Examples of modifications include addition of protecting groups on the amino group (for example, acylation (formylation or acetylation), mesylation, ureation, carbamation, Boc-protection or Fmoc-protection), and esterification (such as ethylation) of the carboxyl group. It may also include modification that can commonly take place in the body, such as phosphorylation, amidation, methylation, esterification and acetylation, as well as modification that takes place during the synthesis process or that is used to facilitate purification, such as biotinylation. Modification such as PEGylation may also be carried out to extend the *in vivo* half-life of the peptide. From the viewpoint of increasing the stability, in particular, the free amino group of the N-terminal amino acid may be protected with a protecting group (such as an acyl group). For example, the free amino group of the N-terminal amino acid may be acetylated or mesylated. In a peptide comprising a sequence in which the carboxyl groups of the aspartic acid residues at position 3 and/or position 8 of PACAP are replaced with tetrazole, the stability can be further increased by acetylation or mesylation of the N-terminus. The C-terminus may be a carboxyl group (-COOH), carboxylate (-COO-), amide ($-CONH_2$) or ester (-COOR), and it may also have a sugar chain addition (see WO2017/027848, for example).

According to one specific aspect, disclosed are peptides 3 to 34 having the sequences listed in Table 1 below, or their modified sequences:

[Table 1]

Table 1

| Peptide Name | N-terminus | Sequence | | C-terminus |
|---|---|---|---|---|
| Peptide 1 | H- | HSDGIFTDSYSRYRKQMAVKKYLAAVL | (SEQ ID No. 2) | $NH_2$ |
| Peptide 2 | Ac- | HSDGIFTDSYSRYRKOMAVKKYLAAVL | (SEQ ID No. 4) | $NH_2$ |
| Peptide 3 | H- | HSTzGIFTDSYSRYRKQMAVKKYLAAVL | (SEQ ID No. 5) | $NH_2$ |
| Peptide 4 | H- | HSDGIFTTzSYSRYRKQMAVKKYLAAVL | (SEQ ID No. 6) | $NH_2$ |
| Peptide 5 | H- | HSTzGIFTTzSYSRYRKQMAVKKYLAAVL | (SEQ ID No. 7) | $NH_2$ |
| Peptide 6 | H- | HSTzGIFTTzSYSRYRKQNIAVKKYLAAVL | (SEQ ID No. 8) | $NH_2$ |
| Peptide 7 | H- | HSTzGIFTTzSYSRYRKQLAVKKYLAAVL | (SEQ ID No. 9) | $NH_2$ |
| Peptide 8 | Ac- | HSTzGIFTTzSYSRYRKQNIAVKKYLAAVL | (SEQ ID No. 10) | $NH_2$ |
| Peptide 9 | Ac- | HSTzGIFTTzSYSRYRKQLAVKKYLAAVL | (SEQ ID No. 11) | $NH_2$ |
| Peptide 10 | Ac- | HSTzGIFTTzSYSRYRKQMAVKKYLAAVL | (SEQ ID No. 12) | $NH_2$ |
| Peptide 11 | Ms- | HSTzGIFTTzSYSRYRKQNIAVKKYLAAVL | (SEQ ID No. 13) | $NH_2$ |
| Peptide 12 | Ms- | HSTzGIFTTzSYSRYRKQLAVKKYLAAVL | (SEQ ID No. 14) | $NH_2$ |

(continued)

| Peptide Name | N-terminus | Sequence | | C-terminus |
|---|---|---|---|---|
| Peptide 13 | Ac- | HSTzGIFTTzSYSRYRKegTzLAVKKYLAAVL | (SEQ ID No. 15) | $NH_2$ |
| Peptide 14 | Ac- | HATzGIFTTzSYSRYRKQLAVKKYLAAVL | (SEQ ID No. 16) | $NH_2$ |
| Peptide 15 | Ac- | HSTzGIFTTzAYSRYRKQLAVKKYLAAVL | (SEQ ID No. 17) | $NH_2$ |
| Peptide 16 | Ac- | HSTzGIFTTzSYARYRKQLAVKKYLAAVL | (SEQ ID No. 18) | $NH_2$ |
| Peptide 17 | Ac- | HSTzGIFTTzSYSRYRKALAVKKYLAAVL | (SEQ ID No. 19) | $NH_2$ |
| Peptide 18 | Ac- | HSTzGIFTTzSYSRYRKQAAVKKYLAAVL | (SEQ ID No. 20) | $NH_2$ |
| Peptide 19 | Ac- | HSTzGIFTTzSYSRYRKQLAAKKYLAAVL | (SEQ ID No. 21) | $NH_2$ |
| Peptide 20 | Ac- | HSTzGIFTTzSYSRYRKQLAVKKYLAAVA | (SEQ ID No. 22) | $NH_2$ |
| Peptide 21 | Ac- | HSTzGIFTTzSYSRYRRQLAVRRYLAAVL | (SEQ ID No. 23) | $NH_2$ |
| Peptide 22 | Ac- | HSTzGIFTTzSYSRYRRQLAVRRYLAAVLGRR | (SEQ ID No. 24) | $NH_2$ |
| Peptide 23 | Ac- | HSTzAIFTTzSYSRYRKQLAVKKYLAAVL | (SEQ ID No. 25) | $NH_2$ |
| Peptide 24 | Ac- | HATzGIFTTzAYSRYRKQLAVKKYLAAVL | (SEQ ID No. 26) | $NH_2$ |
| Peptide 25 | Ac- | HATzGIFTTzSYSRYRKOAAVKKYLAAVL | (SEQ ID No. 27) | $NH_2$ |
| Peptide 26 | Ac- | HSTzGIFTTzAYSRYRKALAVKKYLAAVL | (SEQ ID No. 28) | $NH_2$ |
| Peptide 27 | Ac- | HSTzGIFTTzAYSRYRKQAAVKKYLAAVL | (SEQ ID No. 29) | $NH_2$ |
| Peptide 28 | Ac- | HSTzGIFTTzSYSRYRKAAAVKKYLAAVL | (SEQ ID No. 30) | $NH_2$ |
| Peptide 29 | Ms- | HSTzGIFTTzAYSRYRKQLAVKKYLAAVL | (SEQ ID No. 31) | $NH_2$ |
| Peptide 30 | Ms- | HSTzAIFTTzSYSRYRKQLAVKKYLAAVL | (SEQ ID No. 32) | $NH_2$ |
| Peptide 31 | Ms- | HSTzAIFTTzSYSRYRKQAAVKKYLAAVL | (SEQ ID No. 33) | $NH_2$ |
| Peptide 32 | Ac- | HSTzAIFTTzSYSRYRKQAAVKKYLAAVL | (SEQ ID No. 34) | $NH_2$ |
| Peptide 33 | Ac- | HATzAIFTTzSYSRYRKQAAVKKYLAAVL | (SEQ ID No. 35) | $NH_2$ |
| Peptide 34 | Ac- | HSTzAIFTTzSYSRYRKAAAVKKYLAAVL | (SEQ ID No. 36) | $NH_2$ |

An amino acid in which the carboxyl group of aspartic acid has been replaced with tetrazole has the following structure:

[Chemical Formula 2]

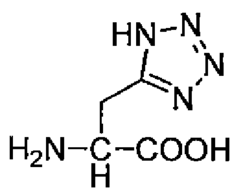

Throughout the present specification, "Tz" will be used as its symbol in the sequences.

An amino acid in which the amide of glutamine has been replaced with tetrazole has the following structure:

[Chemical Formula 3]

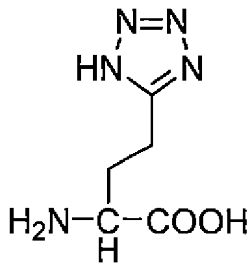

Throughout the present specification, "egTz" will be used as its symbol in the sequences.

The peptide of the invention can be produced by any production method. For example, it can be produced by solid phase synthesis or liquid phase synthesis using the Boc or Fmoc method. It can also be produced by other methods, using a method of transferring nucleic acid coding for the peptide of the invention into a gene, introducing the gene into host cells and synthesizing the peptide in the host cells. Purification after expression can be easily accomplished by a design in which a tag peptide such as a polyhistidine tag is added at the ends of the peptide.

The peptide of the invention also includes its medically acceptable salts. Medically acceptable salts include salts of inorganic acids (for example, hydrochlorides, hydrobromides, sulfates and phosphates), salts of organic acids (for example, methanesulfonates, benzenesulfonates, *p*-toluenesulfonates, formates, acetates, trifluoroacetates, oxalates, citrates, malonates, fumarates, maleates, tartrates, succinates and malates), or salts with bases (for example, ammonium salts, methylpyridinium salts and acetylpyridinium salts). The peptide of the invention also includes hydrates or solvates.

Histidine mesylated at the N-terminus can be produced by the method illustrated by reaction formula 1, or a similar method.

[Chemical Formula 4]

Reaction Scheme 1

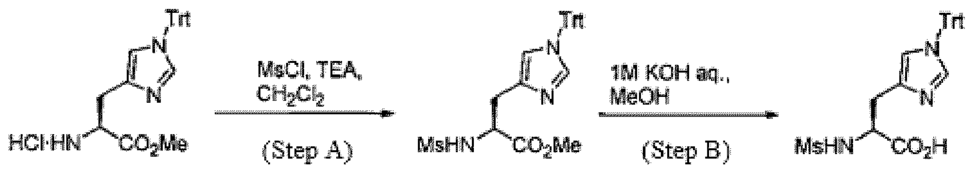


(I) (II) (III)

{wherein Trt represents a trityl group and Me represents a methyl group.}

In the method of reaction formula 1, a compound represented by general formula compound (I) can be reacted with methanesulfonyl chloride in the presence of a base to produce compound (II), after which compound (II) can be subjected to hydrolysis using a base in methanol, to produce compound (III).

For production of compound (II), the base is used at 0.2 to 5 equivalents and preferably 1 to 3 equivalents with respect to compound (I). The base used may be triethylamine, N,N-diisopropylethylamine, pyridine or 4-dimethylaminopyridine, with triethylamine being preferred. Methanesulfonyl chloride is used at 0.1 to 5 equivalents and preferably 1 to 2 equivalents. The solvent is not particularly restricted so long as it does not affect the reaction, and may be tetrahydrofuran, dichloromethane or toluene, for example, with dichloromethane being preferred. The reaction temperature will usually be 1°C to 30°C, and preferably 15°C to 25°C, while the reaction time will usually be 0.5 hour to 12 hours, and preferably 0.5 hour to 2 hours.

For production of compound (III), the base is used at 0.1 to 10 equivalents and preferably 1 to 3 equivalents with respect to compound (II). Bases include lithium hydroxide, sodium hydroxide and potassium hydroxide, with potassium hydroxide being preferred. The solvent may be a mixed solvent of an organic solvent (such as methanol, ethanol, isopropanol, acetonitrile, 1,4-dioxane or tetrahydrofuran) and water, and it is preferably a mixed solvent of methanol and water. The reaction time will differ depending on the reagents or solvent used, but it will usually be 0.5 hour to 12 hours and preferably 0.5 hour to 3 hours. The reaction temperature will also differ depending on the reagents or solvent, but it will usually be 0°C to 100°C and preferably 60°C to 100°C.

The peptide of the invention can exhibit physiological activity similar to PACAP, by binding with PAC1R, VPAC1R and/or VPAC2R. Without being limited to any particular theory, it is thought that the usefulness for preventing or treating

neurotrophic keratitis may be mediated by binding to these receptors. More specifically, the peptide of the invention can inhibit reduction in lacrimal fluid volume caused by neurotrophic keratitis, and can inhibit superficial punctate keratitis. The peptide of the invention can also promote nerve axon elongation. The effect of promoting axon elongation by the peptide of the invention makes it possible to treat trigeminal nerve damage or to regenerate the trigeminal nerve. The peptide of the invention can also treat or prevent neurotrophic keratitis by this activity. The peptide of the invention can further promote nerve axon elongation both *in vivo* and *in vitro.* When axon elongation is to be promoted *in vitro,* neurons are cultured in medium containing the peptide of the invention. The neuron culturing method can be carried out by any procedure that is known in the technical field, using a neuron culture medium that is widely known in the technical field.

The present invention relates to peptide as claimed that can be used in a pharmaceutical composition for treatment or prevention of neurotrophic keratitis, comprising a therapeutically effective dose of the aforementioned peptide. The pharmaceutical composition can treat neurotrophic keratitis by physiological action of PACAP, by administration to a patient, or it can prevent neurotrophic keratitis by administration to a patient with the potential for developing the disease. Here, "treatment" means that after onset of a disorder or disease, worsening of the condition is prevented, its progression is delayed, or the current condition is maintained, alleviated or reversed, while "prevention" means that onset of a disorder or disease is prevented before onset. A patient with the potential for developing neurotrophic keratitis may be, for example, a patient suffering from corneal herpes viral infection, eye injury, corneal surgery or diabetes.

The peptide of the invention, or the pharmaceutical composition comprising the peptide, may be administered to a patient to be treated by either parenteral administration or oral administration. Oral administration may be sublingual, intraoral or oral administration. Examples of parenteral administration include administration by intravenous, intraarterial, subcutaneous, local, intraperitoneal, intramuscular, nasal, transdermal, transmucosal, intrameningeal, perrectal, intramuscular, intracerebral, intrameningeal, subarachnoid, intradural, epidural, eye drop, ear drop, nasal drop or intraocular routes. Intraocular routes include, more specifically, subconjunctival, sub-tenon and intravitreal routes. A pharmaceutical composition comprising the peptide of the invention may be prepared in an appropriate dosage form for the route of administration, and may be an eye drop, injection, powdered drug, infusion preparation, granules, tablets or suppository, for example, but for parenteral administration it is preferably an eye drop, injection, infusion preparation, or a powdered drug for preparation prior to use. A formulation for intraocular administration may be an intravitreal injection, subconjunctival injection or sub-tenon injection, for example. Such formulations may also contain various pharmaceutically acceptable adjuvants, i.e. additives such as carriers or other auxiliary agents, which include stabilizers, antiseptic agents, soothing agents and emulsifiers. They may also be used in combination with other drugs that have neuroprotective effects, inflammationsuppressing effects or exocrine gland secretion effects.

The examples of the invention described below are intended to serve merely as illustration and do not limit the technical scope of the invention. The technical scope of the invention is limited solely by the Claims.

EXAMPLES

Example 1: Peptide synthesis 1

Synthesis of Ms-His(Trt)-OMe

[Chemical Formula 5]

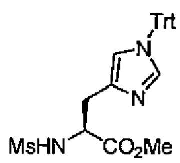

After adding methanesulfonyl chloride (314.7 mg, 2.7 mmol) dissolved in N,N-diisopropylethylamine (1.0 mL, 5.7 mmol) and dichloromethane (2.0 mL), to a solution of N-im-trityl-L-histidine methyl ester hydrochloride (2.02 g, 4.5 mmol) in dichloromethane (40 mL) and reacting at 25°C for 2 hours and 40 minutes, methanesulfonyl chloride (174.7 mg, 1.5 mmol) dissolved in dichloromethane (1.0 mL) was further added and reaction was carried out at 25°C for 1 hour. A saturated sodium hydrogencarbonate aqueous solution was then added to stop the reaction, extraction was performed with ethyl acetate, the obtained organic layer was washed with a 10% citric acid aqueous solution, a saturated sodium hydrogencarbonate aqueous solution and brine, the organic layer was dried over anhydrous sodium sulfate, and the solvent was distilled off under reduced pressure. Since the reaction did not proceed to completion, dichloromethane (15

mL) was added to the obtained residue, and then a solution of triethylamine (1.0 mL, 7.2 mmol) and methanesulfonyl chloride (326.8 mg, 2.9 mmol) in dichloromethane (1.0 mL) was added and the mixture was stirred at room temperature for 1 hour. A saturated sodium hydrogencarbonate aqueous solution was then added to stop the reaction, extraction was performed with ethyl acetate, the obtained organic layer was washed with a 10% citric acid aqueous solution, a saturated sodium hydrogencarbonate aqueous solution and brine, the organic layer was dried over anhydrous sodium sulfate, and the solvent was distilled off under reduced pressure to obtain a pale yellow solid (2.35 g, >100%). The obtained solid was used in the following reaction without purification.
$^1$H-NMR (400 MHz, $CDCl_3$) δ 7.38 (1H, d, J = 1.2 Hz), 7.34-7.31 (9H, m), 7.12-7.08 (6H, m), 6.57 (1H, d, J = 2.4 Hz), 6.30 (1H, d, J = 8.4 Hz), 4.40 (1H, dt, J = 8.4, 5.2 Hz), 3.65 (3H, s), 3.05 (2H, d, J = 5.2 Hz), 2.97 (3H, s).

Synthesis of Ms-His(Trt)-OH

[Chemical Formula 6]

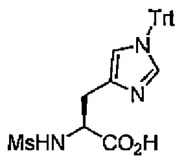

A 1 M potassium hydroxide aqueous solution (9.0 mL, 9.0 mmol) was added to a methanol solution (15 mL) containing Ms-His-OMe (2.21 g, 4.5 mmol), and the mixture was refluxed for 1.5 hours. The reaction mixture was cooled to room temperature, acidified to pH 5 with a 10% citric acid aqueous solution, and extracted with dichloromethane. The obtained organic layer was dried over sodium sulfate and the solution was distilled off under reduced pressure, after which dichloromethane and hexane were added to the residue to precipitate a white solid, which was filtered (2.32 g, >100%).
$^1$H-NMR (400 MHz, $CDCl_3$) δ 7.84 (1H, s), 7.31-7.33 (9H, m), 7.12-7.09 (6H, m), 6.82 (1H, s), 4.13 (1H, m), 3.34 (1H, dd, J = 14.8, 3.6 Hz), 3.17 (1H, dd, J = 14.8, 6.8 Hz), 2.90 (3H, s).

Example 2: Peptide synthesis 2

The peptide used in the test was synthesized using a peptide synthesizer (model: PSSM-8 by Shimadzu Corp.), using solid phase synthesis by the Fmoc method. The non-natural amino acids used in the solid phase synthesis were Fmoc-TZ-OH, Fmoc-TZ (trt)-OH and Fmoc-egTZ (trt)-OH, purchased from Astatech Inc. Peptides 1 to 34 having the following sequences were synthesized, and the molecular weights of the synthetic peptides were subjected to mass spectrometry (MALDI TOF). As shown in Table 2, all of the measured values matched theoretical values.

[Table 2]

Table 2

| Peptide Name | N-terminus | Sequence | | C-terminus | Theoretical Value | Measured Value |
|---|---|---|---|---|---|---|
| Peptide 1 | H- | HSDGIFTDSYSRYRKQMAVKKYLAAVL | (SEQ ID No. 2) | $NH_2$ | 3146.66 | 3147.0 |
| Peptide 2 | Ac- | HSDGIFTDSYSRYRKQMAVKKYLAAVL | (SEQ ID No. 4) | $NH_2$ | 3188.67 | 3188.4 |
| Peptide 3 | H- | HSTzGIFTDSYSRYRKQMAVKKYLAAVL | (SEQ ID No. 5) | $NH_2$ | 3170.68 | 3171.1 |
| Peptide 4 | H- | HSDGIFTTzSYSRYRKQMAVKKYLAAVL | (SEQ ID No. 6) | $NH_2$ | 3170.68 | 3170.8 |
| Peptide 5 | H- | HSTzGIFTTzSYSRYRKOMAVKKYLAAVL | (SEQ ID No. 7) | $NH_2$ | 3194.70 | 3194.2 |
| Peptide 6 | H- | HSTzGIFTTzSYSRYRKONIAVKKYLAAVL | (SEQ ID No. 8) | $NH_2$ | 3176.75 | 3177.0 |
| Peptide 7 | H- | HSTzGIFTTzSYSRYRKQLAVRKYLAAVL | (SEQ ID No. 9) | $NH_2$ | 3176.75 | 3176.9 |
| Peptide 8 | Ac- | HSTzGIFTTzSYSRYRKQNIAVKKYLAAVL | (SEQ ID No. 10) | $NH_2$ | 3218.76 | 3219.2 |
| Peptide 9 | Ac- | HSTzGIFTTzSYSRYRKQLAVKKYLAAVL | (SEQ ID No. 11) | $NH_2$ | 3218.76 | 3218.2 |
| Peptide 10 | Ac- | HSTzGIFTTzSYSRYRKQMAVKKYLAAVL | (SEQ ID No. 12) | $NH_2$ | 3236.71 | 3236.7 |
| Peptide 11 | Ms- | HSTzGIFTTzSYSRYRKQNIAVKKYLAAVL | (SEQ ID No. 13) | $NH_2$ | 3254.72 | 3255.0 |
| Peptide 12 | Ms- | HSTzGIFTTzSYSRYRKQLAVKKYLAAVL | (SEQ ID No. 14) | $NH_2$ | 3254.72 | 3254.4 |
| Peptide 13 | Ac- | HSTzGIFTTzSYSRYRKegTzLAVKKYLAAVL | (SEQ ID No. 15) | $NH_2$ | 3243.76 | 3243.7 |
| Peptide 14 | Ac- | HATzGIFTTzSYSRYRKOLAVKXYLAAVL | (SEQ ID No. 16) | $NH_2$ | 3202.76 | 3202.8 |
| Peptide 15 | Ac- | HSTzGIFTTzAYSRYRKQLAVKKYLAAVL | (SEQ ID No. 17) | $NH_2$ | 3202.76 | 3202.4 |
| Peptide 16 | Ac- | HSTzGIFTTzSYARYRKQLAVKKYLAAVL | (SEQ ID No. 18) | $NH_2$ | 3202.76 | 3202.8 |
| Peptide 17 | Ac- | HSTzGIFTTzSYSRYRKALAVKKYLAAVL | (SEQ ID No. 19) | $NH_2$ | 3161.73 | 3161.4 |
| Peptide 18 | Ac- | HSTzGIFTTzSYSRYRKQAAVKKYLAAVL | (SEQ ID No. 20) | $NH_2$ | 3176.71 | 3176.6 |
| Peptide 19 | Ac- | HSTzGIFTTzSYSRYRKQLAAKKYLAAVL | (SEQ ID No. 21) | $NH_2$ | 3190.72 | 3190.2 |
| Peptide 20 | Ac- | HSTzGIFTTzSYSRYRKQLAVNKYLAAVA | (SEQ ID No. 22) | $NH_2$ | 3176.71 | 3176.4 |
| Peptide 21 | Ac- | HSTzGIFTTzSYSRYRRGLAVRRYLAAVL | (SEQ ID No. 23) | $NH_2$ | 3302.11 | 3303.1 |
| Peptide 22 | Ac- | HSTzGIFTTzSYSRYRRQLAVRRYLAAVLGRR | (SEQ ID No. 24) | $NH_2$ | 3672.00 | 3672.5 |
| Peptide 23 | Ac- | HSTzAIFTTzSYSRYRKQLAVKKYLAAVL | (SEQ ID No. 25) | $NH_2$ | 3232.77 | 3232.8 |
| Peptide 24 | Ac- | HATzGIFTTzAYSRYRKQLAVKKYLAAVL | (SEQ ID No. 26) | $NH_2$ | 3186.77 | 3186.3 |
| Peptide 25 | Ac- | HATzGIFTTzSYSRYRKGAAVKKYLAAVL | (SEQ ID No. 27) | $NH_2$ | 3160.72 | 3160.2 |

(continued)

| Peptide Name | N-terminus | Sequence | C-terminus | Theoretical Value | Measured Value |
|---|---|---|---|---|---|
| Peptide 26 | Ac- | HSTzGIFTTzAYSRYRKALAVKKYLAAVL (SEQ ID No. 28) | $NH_2$ | 3145.74 | 3145.3 |
| Peptide 27 | Ac- | HSTzGIFTTzAYSRYRKQAAVKKYLAAVL (SEQ ID No. 29) | $NH_2$ | 3160.71 | 3160.3 |
| Peptide 28 | Ac- | HSTzGIFTTzSYSRYRKAAAVKKYLAAVL (SEQ ID No. 30) | $NH_2$ | 3119.69 | 3119.3 |
| Peptide 29 | Ms- | HSTzGIFTTzAYSRYRKQLAVKKYLAAVL (SEQ ID No. 31) | $NH_2$ | 3238.73 | 3238.7 |
| Peptide 30 | Ms- | HSTzALFTTzSYSRYRKQLAVKKYLAAVL (SEQ ID No. 32) | $NH_2$ | 3268.74 | 3268.6 |
| Peptide 31 | Ms- | HSTzAIFTTzSYSRYRKQAAVKKYLAAVL (SEQ ID No. 33) | $NH_2$ | 3226.69 | 3226.3 |
| Peptide 32 | Ac- | HSTzAIFTTzSYSRYRKQAAVKKYLAAVL (SEQ ID No. 34) | $NH_2$ | 3190.72 | 3191.1 |
| Peptide 33 | Ac- | HATzAIFTTzSYSRYRKQAAVKKYLAAVL (SEQ ID No. 35) | $NH_2$ | 3174.73 | 3174.4 |
| Peptide 34 | Ac- | HSTzAIFTTzSYSRYRKAAAVKKYLAAVL (SEQ ID No. 36) | $NH_2$ | 3133.70 | 3134.0 |

Example 3: Peptide stability test 1

[Preparation of measuring sample]

Peptides 1 and 3 to 5 synthesized in Example 2 were weighed and dissolved in phosphate buffer (pH 7.0) to prepare 1.0 mM peptide solutions. The 1.0 mM peptide solutions were then diluted with phosphate buffer to 100 μM. After filtering with Chromatdisk (product of Merck Millipore, Millex-GV, 0.22 μm), each filtrate was dispensed into an LC vial (Waters Deactivated Qsert Vial). The prepared peptide solutions were incubated for one month or 2 months in a 40°C thermostatic bath to obtain stored samples. A simultaneously prepared sample among the peptide solutions that was not stored was used as a standard sample (initial sample). The standard sample and the stored samples were stored at -30°C until sample analysis.

[Moisture permeability]

Using the total weight of the aqueous peptide solution and storage vessel as the specimen weight, measurements were recorded as the specimen weights before storage and the specimen weights after storage under different storage conditions. The empty weight of the storage vessel was also measured, and the moisture permeability was determined by the following formula.

[Mathematical Formula 1]

$$\text{Vessel moisture permeability (\%)} = \frac{\text{Specimen weight before storage (g)} - \text{specimen weight after storage (g)}}{\text{Specimen weight before storage (g)} - \text{storage vessel weight (g)}} \times 100$$

After stirring the standard sample and the stored samples with a vortex mixer, each peptide solution was transferred to an HPLC vial (Deactivated Qsert vial by Waters Co.). Reverse-phase HPLC (HPLC system: Prominence by Shimadzu Corp.) was conducted under the conditions shown in Table 3 below for analysis of the peptide solutions, and chromatograms were obtained.

[HPLC analysis conditions 1]

Column: XSelect CSH C18 by Waters Co., 5 μm, 4.6 × 250 mm
Guard column: XSelect CSH C18 by Waters Co., 5 μm, 4.6 × 20 mm Guard Cartridge
Detection wavelength: 220 nm
Mobile phase A: 0.1% formic acid aqueous solution
Mobile phase B: 0.1% acetonitrile formate solution
Measuring time: 30 minutes
Measuring sample injection rate: 50 μL
Flow rate: 1.0 mL/min
Sample cooler: 4°C
Column temperature: 40°C

Mobile phase delivery: The mixing ratio of mobile phase A and mobile phase B was varied as shown in Table 3 for linear concentration gradient control.

[Table 3]

Table 3

| Time after sample injection (min) | Mobile phase A (vol%) | Mobile phase B (vol%) |
|---|---|---|
| 0-30.0 | 90→75 | 10→25 |
| 30.0-30.5 | 75→0 | 25→100 |

(continued)

| Time after sample injection (min) | Mobile phase A (vol%) | Mobile phase B (vol%) |
|---|---|---|
| 30. 5-36.5 | 0 | 100 |
| 36.5-37.0 | 0→90 | 100→10 |
| 37.0-49.5 | 90 | 10 |

The peak area value for the peptide in the chromatogram was calculated, and the remaining rate (remaining rate before correction for water) was calculated using formula (2). The remaining rate after correction for water was also calculated from the remaining rate before correction for water using formula (3), taking into account the moisture permeability of the vessel.
[Mathematical Formula 2]

$$\text{Remaining rate before correction for water (\%)} = \frac{\text{Peptide peak area of stored sample}}{\text{Peptide peak area of standard sample}} \times 100 \quad (2)$$

[Mathematical Formula 3]

$$\text{Remaining rate after correction for water (\%)} = \frac{\text{Remaining rate before correction for water (\%)} \times (100\% - \text{vessel moisture permeability (\%)})}{100} \quad (3)$$

Table 4 shows the remaining rate after correction for water for each peptide among the stored samples.

[Table 4]

Table 4

| Peptide No. | 40°C, 1 month preservation | 40°C, 2 months preservation |
|---|---|---|
| Peptide 1 | 37.4% | 21.3% |
| Peptide 3 | 76.5% | 60.7% |
| Peptide 4 | 42.8% | 28.2% |
| Peptide 5 | 94.7% | 85.6% |

Peptide 1 (PACAP) had low stability, at 37.4% after storage at 40°C for one month and 21.3% after storage at 40°C for 2 months. Peptide 3, which had only the carboxyl group of the aspartic acid side chain at the 3rd residue of peptide 1 replaced with tetrazole, exhibited higher stability than peptide 1, while peptide 4, which had only the carboxyl group of the aspartic acid side chain at the 8th residue replaced with tetrazole, also had increased stability compared to peptide 1. However, peptide 5, which had the carboxyl groups of the aspartic acid side chains of the 3rd and 8th residues of peptide 1 replaced with tetrazole, had further increased stability over peptide 3 and peptide 4, thus demonstrating that for increased PACAP stability it is more effective to have two tetrazole substitutions than a single tetrazole substitution.

Example 4: Peptide stability test 2

Peptides 1, 2 and 6 to 9 synthesized in Example 2 were weighed and dissolved in Tris buffer (pH 7.0) to prepare 1.0 mM peptide solutions. Each solution was filtered with a Chromatdisk (product of Merck Millipore, Millex-GV, 0.22 μm). The filtrate was diluted to 100 μM with Tris buffer (pH 7.0) and dispensed into a tube (Protein LoBind Tube by Eppendorf Co.). The prepared peptide solutions were incubated for 1 week or 2 weeks in a 60°C thermostatic bath to obtain stored samples. A simultaneously prepared sample among the peptide solutions that was not stored was used as a standard sample (initial sample). The standard sample and the stored samples were stored at -30°C until sample analysis.
Each stored sample was measured under the following HPLC analysis conditions 2, and the remaining rate after correction for water for each peptide among the stored samples was calculated in the same manner as Example 3. The results are shown in Table 6.

[HPLC analysis conditions 2]

Column: XSelect CSH C18 by Waters Co., 5 μm, 4.6 × 250 mm
Guard column: XSelect CSH C18 by Waters Co., 5 μm, 4.6 × 20 mm Guard Cartridge
Detection wavelength: 220 nm
Mobile phase A: 0.1% formic acid aqueous solution
Mobile phase B: 0.1% acetonitrile formate solution
Measuring time: 20 minutes
Measuring sample injection rate: 50 μL
Flow rate: 1.0 mL/min
Column temperature: 40°C
Sample cooler: 25°C
Mobile phase delivery: The mixing ratio of mobile phase A and mobile phase B was varied as shown in Table 5 for linear concentration gradient control.

[Table 5]

Table 5

| Time after sample injection (m in) | Mobile phase A (vol%) | Mobile phase B (vol%) |
|---|---|---|
| 0 - 20.0 | 85 → 75 | 15 → 25 |
| 20.0 - 20.1 | 75 → 0 | 25 → 100 |
| 20.1 - 24. 5 | 0 | 100 |
| 24.5 - 25.0 | 0 → 85 | 100 → 15 |
| 25.0 - 40.0 | 85 | 15 |

[Table 6]

Table 6

| Peptide No. | 60°C, 1 week preservation | 60°C, 2 weeks preservation |
|---|---|---|
| Peptide 1 | 26.6% | 15.8% |
| Peptide 2 | 28.7% | 17.2% |
| Peptide 6 | 91.3% | 82.4% |
| Peptide 7 | 91.7% | 84.8% |
| Peptide 8 | 98.1% | 92.9% |
| Peptide 9 | 98.4% | 92.8% |

Peptide 1 (PACAP) had very poor stability, at 26.6% after storage for 1 week at 60°C and 15.8% after storage for 2 weeks, while peptide 2, which had the N-terminus of PACAP acetylated, exhibited only equivalent stability. Peptides 6 and 7, on the other hand, which had the carboxyl groups of the aspartic acids at position 3 and position 8 replaced with tetrazole (peptide 6 also having the methionine at position 17 replaced by norleucine (indicated as "Nl" in the sequence) and peptide 7 further having the methionine at position 17 replaced by leucine), had drastically increased stability, at 91.3% and 91.7% after storage for 1 week at 60°C, and 82.4% and 84.8% after storage for 2 weeks. Peptide 8 and 9, which had the N-termini of peptides 6 and 7 acetylated, had further increased stability, at 98.1% and 98.4% after storage for 1 week at 60°C, and 92.9% and 92.8% after storage for 2 weeks. An accelerated test for 1 week and 2 weeks at 60°C corresponds respectively to approximately 1 year and 2 years at room temperature (25°C). Therefore, peptides 8 and 9 are expected to be stable

(remaining rate of 90% or higher) for 2 years at room temperature.

Example 5: Peptide stability test 3

Peptides 12 to 25, 27 and 28 synthesized in Example 2 were weighed and dissolved in Tris buffer (pH 7.0) to prepare 1.0 mM peptide solutions. Each solution was filtered with a Chromatdisk (product of Merck Millipore, Millex-GV, 0.22 μm). The filtrate was diluted to 100 μM with Tris buffer (pH 7.0) and dispensed into a tube (Protein LoBind Tube by Eppendorf Co.). The prepared peptide solutions were incubated for one week or 2 weeks in a 60°C thermostatic bath to obtain stored samples. A simultaneously prepared sample among the peptide solutions that was not stored was used as a standard sample (initial sample). The standard sample and the stored samples were stored at -30°C until sample analysis.

The remaining rate after correction for water for each peptide among the stored samples was calculated in the same manner as Example 4, and the results are shown in Table 7.

[Table 7]

Table 7

| Peptide No. | 60°C, 1 week preservation | 60°C, 2 weeks preservation |
|---|---|---|
| Peptide 12 | 97.0% | 93.6% |
| Peptide 13 | 97.4% | 95.4% |
| Peptide 14 | 96.7% | 94.5% |
| Peptide 15 | 97.3% | 94.9% |
| Peptide 16 | 98.1% | 93.5% |
| Peptide 17 | 97.8% | 93.4% |
| Peptide 18 | 96.7% | 90.6% |
| Peptide 19 | 95.8% | 92.7% |
| Peptide 20 | 93.9% | 91.8% |
| Peptide 21 | 97.8% | 93.5% |
| Peptide 22 | 98.2% | Not Measured |
| Peptide 23 | 96.5% | 93.1% |
| Peptide 24 | 98.1% | 94.3% |
| Peptide 25 | 95.8% | 91.2% |
| Peptide 27 | 96.4% | 93.5% |
| Peptide 28 | 94.6% | 90.5% |

Peptide 12 (a peptide having the N-terminal acetyl group of peptide 9 replaced with a mesyl group) exhibited similar stability to peptide 9, and therefore high stability was exhibited even with N-terminal mesyl group substitution. Peptide 13 (a peptide having the glutamine side chain of the 16th residue of peptide 9 replaced with tetrazole) exhibited similar stability to peptide 9, indicating that high stability is maintained even with a peptide having the glutamine side chain replaced with tetrazole. High stability was also maintained with peptides 14 to 25, peptide 27 and peptide 28, which had alanine or arginine substituting or added at arbitrary residues of peptide 9.

Example 5-2: Peptide stability test 3

Peptides 29 to 34 synthesized in Example 2 were weighed and dissolved in Tris buffer (pH 7.0) to prepare 1.0 mM peptide solutions. Each solution was filtered with a Chromatdisk (product of Merck Millipore, Millex-GV, 0.22 μm). The filtrate was diluted to 100 μM with Tris buffer (pH 7.0) and dispensed into a tube (Protein LoBind Tube by Eppendorf Co.). The prepared peptide solutions were incubated for 2 weeks in a 60°C thermostatic bath to obtain stored samples. A simultaneously prepared sample among the peptide solutions that was not stored was used as a standard sample (initial

sample). The standard sample and the stored samples were stored at -30°C until sample analysis.

The remaining rate after correction for water for each peptide among the stored samples was calculated in the same manner as Example 4, and the results are shown in Table 7-2.

[Table 7-2]

Table 7.2

| Peptide No. | 60°C, 2 weeks preservation |
|---|---|
| Peptide 29 | 91.9% |
| Peptide 30 | 92.2% |
| Peptide 31 | 901% |
| Peptide 32 | 88.7% |
| Peptide 33 | 85.3% |
| Peptide 34 | 96.9% |

Peptide 29 (a peptide having the N-terminal acetyl group of peptide 15 replaced with a mesyl group) exhibited similar stability to peptide 15, and therefore high stability was exhibited even with N-terminal mesyl group substitution. Peptide 30 (a peptide having the N-terminal acetyl group of peptide 23 replaced with a mesyl group) exhibited similar stability to peptide 23, and therefore high stability was exhibited even with N-terminal mesyl group substitution. High stability was also maintained with peptides 32 to 34, which had alanine substituting at arbitrary residues of peptide 9. Peptide 31 (a peptide having the N-terminal acetyl group of peptide 32 replaced with a mesyl group) exhibited similar stability to peptide 32.

Example 6: Peptide stability test 4

Peptides 10 and 11 synthesized in Example 2 were weighed and dissolved in phosphate buffer (pH 7.0) to prepare 1.0 mM peptide solutions. Each solution was filtered with a Chromatdisk (product of Merck Millipore, Millex-GV, 0.22 μm). Each filtrate was diluted to 100 μM with phosphate buffer (pH 7.0) and dispensed into a tube (Protein LoBind Tube by Eppendorf Co.). The prepared peptide solutions were incubated for one month or 2 months in a 40°C thermostatic bath to obtain stored samples. A simultaneously prepared sample among the peptide solutions that was not stored was used as a standard sample (initial sample). The standard sample and the stored samples were stored at -30°C until sample analysis.

The remaining rate after correction for water for each peptide among the stored samples was measured in the same manner as Example 4, and the results are shown in Table 8.

[Table 8]

Table 8

| Peptide No. | 40°C, 1 month preservation | 40°C, 2 months preservation |
|---|---|---|
| Peptide 10 | 94.0% | 86.8% |
| Peptide 11 | 94.2% | 88.8% |

The results for peptide 10 having an N-terminal acetyl group and peptide 11 having a N-mesyl group demonstrated that the peptide is stabilized to the same degree whether the N-terminal substituent is an acetyl group or an N-mesyl group.

Example 7: Peptide stability test 5

Peptide 26 synthesized in Example 2 was weighed and dissolved in Tris buffer (pH 7.0) to prepare a 1.0 mM

peptide solution. The solution was filtered with a Chromatdisk (product of Merck Millipore, Millex-GV, 0.22 μm). The filtrate was diluted to 100 μM with Tris buffer (pH 7.0) and dispensed into a tube (Protein LoBind Tube by Eppendorf Co.). The prepared peptide solution was incubated for one week or 2 weeks in a 60°C thermostatic bath to obtain stored samples. A simultaneously prepared sample among the peptide solutions that was not stored was used as a standard sample (initial sample). The standard sample and the stored sample were stored at -30°C until sample analysis.

The stored sample was measured under the following HPLC analysis conditions 3, and the remaining rate after correction for water for the peptide in the stored sample was calculated in the same manner as Example 3. The results are shown in Table 10.

[HPLC analysis conditions 3]

Column: XSelect CSH C18 by Waters Co., 5 μm, 4.6 × 250 mm
Guard column: XSelect CSH C18 by Waters Co., 5 μm, 4.6 × 20 mm Guard Cartridge
Detection wavelength: 220 nm
Mobile phase A: 0.1% formic acid aqueous solution
Mobile phase B: 0.1% acetonitrile formate solution
Measuring time: 20 minutes
Measuring sample injection rate: 50 μL
Flow rate: 1.0 mL/min
Column temperature: 40°C
Sample cooler: 25°C
Mobile phase delivery: The mixing ratio of mobile phase A and mobile phase B was varied as shown in Table 9 for linear concentration gradient control.

[Table 9]

Table 9

| Time after sample injection (m in) | Mobile phase A (vol%) | Mobile phase B (vol%) |
|---|---|---|
| 0 - 20.0 | 82.5 → 72.5 | 17.5 → 27.5 |
| 20.0 - 20. 1 | 72.5 → 0 | 27.5 → 100 |
| 20.1 - 24. 5 | 0 | 100 |
| 24.5 - 25.0 | 0 → 82.5 | 100 → 17.5 |
| 25.0 - 40.0 | 82.5 | 17.5 |

[Table 10]

Table 10

| Peptide No. | 60°C, 1 week preservation | 60°C, 2 weeks preservation |
|---|---|---|
| Peptide 26 | 98. 0% | 94.8% |

Peptide 26 exhibited similar high stability.

Example 7: cAMP assay of PACAP27 and its stabilized peptide

[Cell culturing]

CHO-K1 cells (PAC1 or VPAC1 receptor high-expressing cell line: purchased from DiscoveRx Co.) that had been

mitomycin-treated and frozen were prepared with Cell plating reagent (product of DiscoveRx) at 1.35 × $10^4$ cells/100 μl/well, and then seeded in a 96-well culture plate. The cells were cultured for 18 to 24 hours at 37°C in a 5% $CO_2$ incubator, adhering the cells to the plate.

[Reagent preparation]

Powders of peptides 1, 2 and 6 to 9 synthesized in Example 2 were dissolved in water to 0.1 mM, and then diluted to 20 μM with Cell assay buffer (DiscoveRx) (containing 0.5 mM IBMX and 0.001% BSA). A 5-fold dilution series was then prepared with the same Cell assay buffer and used in an assay.

[cAMP assay]

The cAMP assay was carried out using a Hit Hunter cAMP Assay for Biologics kit (product of DiscoveRx, Cat. No. 90-0075LM25), according to the included kit instructions. The cAMP antibody solution and the solutions of peptides 1, 2 and 6 to 9 diluted to different concentrations were combined to prepare peptide-cAMP antibody liquid mixtures. Next, the culture medium was removed from the CHO-K1 cell culture plate, washing was performed with PBS, and the peptide-cAMP antibody liquid mixtures were added to the cells and incubated for 30 minutes under a 5% $CO_2$ atmosphere at 37°C. A working detection solution was then added and the culture plate was shielded from light with aluminum foil and incubated at 25°C for 1 hour. After incubation, Solution A was added and the culture plate was shielded from light with aluminum foil and then incubated at 25°C for 3 hours. Finally, the chemiluminescence signal was detected using a GloMax detector (Promega) under conditions of luminescence and an integration time of 1 sec. The obtained value for the Relative Luminescence Unit (RLU) was analyzed with GraphPad Prism Ver 6.05 (product of Graph Pad) and the $EC_{50}$ value was calculated for each peptide. The results for the $EC_{50}$ value for the cAMP inducing effect of each peptide with the PAC1R or VPAC1R high-expressing cell line are shown in Fig. 1, Fig. 2 and Table 11.

[Table 11]

Table 11

| Peptide No. | $EC_{50}$ (PAC1R) | $EC_{50}$ (VPAC1R) |
|---|---|---|
| Peptide 1 | 0.052 nM | 0.097 nM |
| Peptide 2 | 0.032 nM | 0.062 nM |
| Peptide 6 | 0.079 nM | 0.093 nM |
| Peptide 7 | 0.112 nM | 0.114 nM |
| Peptide 8 | 0.072 nM | 0.046 nM |
| Peptide 9 | 0.063 nM | 0.043 nM |

The synthesized peptides 1, 2 and 6 to 9 exhibited cAMP inducing ability in the PAC1R and VPAC1R high-expressing cells, and their $EC_{50}$ values were approximately the same as the native peptide (peptide 1: PACAP27).

To summarize the results in Table 6 and Table 11, the peptides of the invention (peptides 6 to 9) have extremely improved stability in aqueous solution compared to PACAP, while also maintaining physiological activity equivalent to that of PACAP. In particular, because they have storage life exceeding 2 years at room temperature, they allow liquid formulations to be developed as products such as vials, ampules and eye drops.

Example 8: PACAP27 stabilized peptide cAMP assay 2

[Reagent preparation]

Powders of peptides 3 to 5 and 10 to 28 synthesized in Example 2 were dissolved in water to 0.1 mM, and then diluted to 20 μM with Cell assay buffer (DiscoveRx) (containing 0.5 mM IBMX and 0.001% BSA). A 5-fold dilution series was then prepared with the same Cell assay buffer and used in an assay.

The $EC_{50}$ values of peptides 3 to 5 and 10 to 28 were calculated by the same method as Example 7. The results for the $EC_{50}$ value for the cAMP inducing effect of each peptide with the PAC1 or VPAC1 high-expressing cell line are shown in

Table 12.

[Table 12]

Table 12

| Peptide No. | $EC_{50}$ (PAC1R) | $EC_{50}$ (VPAC1R) |
|---|---|---|
| Peptide 3 | 0.060 nM | 0.101 nM |
| Peptide 4 | 0.040 nM | 0.053 nM |
| Peptide 5 | 0.033 nM | 0.064 nM |
| Peptide 10 | 0.076 nM | 0.101 nM |
| Peptide 11 | 0.038 nM | 0.190 nM |
| Peptide 12 | 0.023 nM | 0.087 nM |
| Peptide 13 | 0.132 nM | 0.072 nM |
| Peptide 14 | 0.074 nM | 0.099 nM |
| Peptide 15 | 0.061 nM | 0.149 nM |
| Peptide 16 | 0.330 nM | 0.065 nM |
| Peptide 17 | 0.099 nM | 0.132 nM |
| Peptide 18 | 0.061 nM | 0.033 nM |
| Peptide 19 | 0.094 nM | 0.044 nM |
| Peptide 20 | 0.080 nM | 0.040 nM |
| Peptide 21 | 0.102 nM | 0.268 nM |
| Peptide 22 | 0.130 nM | 0.457 nM |
| Peptide 23 | 0.079 nM | 0.072 nM |
| Peptide 24 | 0.083 nM | 0.088 nM |
| Peptide 25 | 0.088 nM | 0.028 nM |
| Peptide 26 | 0.066 nM | 0.075 nM |
| Peptide 27 | 0.060 nM | 0.044 nM |
| Peptide 28 | 0.084 nM | 0.029 nM |

The peptides of the invention (peptides 3 to 5 and 10 to 28) have extremely improved stability in aqueous solution compared to PACAP, while also maintaining physiological activity equivalent to that of PACAP.

Example 8-2: PACAP27 stabilized peptide cAMP assay 2

[Reagent preparation]

Powders of peptides 29 to 34 synthesized in Example 2 were each dissolved in water to 0.1 mM, and then diluted to 20 μM with Cell assay buffer (DiscoveRx) (containing 0.5 mM IBMX and 0.001% BSA). A 5-fold dilution series was then prepared with the same Cell assay buffer and used in an assay.

The $EC_{50}$ values of peptides 29 to 34 were calculated by the same method as Example 7. The results for the $EC_{50}$ value for the cAMP inducing effect of each peptide with the PAC1 or VPAC1 high-expressing cell line are shown in Table 12-2.

[Table 12-2]

| Peptide No. | $EC_{50}$ (PAC1R) | $EC_{50}$ (VPAC1R) |
|---|---|---|
| Peptide 29 | 0.025 nM | 0.090 nM |
| Peptide 30 | 0.032 nM | 0.057 nM |
| Peptide 31 | 0.034 nM | 0.035 nM |
| Peptide 32 | 0.148 nM | 0.076 nM |
| Peptide 33 | 0.089 nM | 0.143 nM |
| Peptide 34 | 0.210 nM | 0.089 nM |

The peptides of the invention (peptides 29 to 34) have extremely improved stability in aqueous solution compared to PACAP, while also maintaining physiological activity equivalent to that of PACAP.

Example 9: Evaluation of PACAP27 and its stabilized peptide in neurotrophic keratitis model

A rat model was prepared, by subcutaneous administration of capsaicin in the dorsal region of rats, which elicited corneal nerve injury and attenuated corneal sensation while also leading to decreased lacrimal fluid volume and superficial punctate keratitis (SPK) (NPL 3: Investigative Ophthalmology & Visual Science(2012), vol. 53, No. 13, p.8280-8287). The rats were given PACAP27 and its stabilized peptide eye drops, and the inhibiting effect on corneal damage was examined. The specific experiment method was as follows.

[Capsaicin administration]

Capsaicin (product of Sigma) was dissolved in PBS containing 10% EtOH and 10% Tween80, and subcutaneously administered to the dorsal region of 4-day-old male Wistar/ST rats at a dose of 50 mg/kg·b.w.

[Preparation of eye drops]

The base was prepared by dissolving 0.3 g of trishydroxymethylaminomethane (Nacalai Tesque, Inc.) and 4.4 g of D(-)-mannitol (Nacalai Tesque, Inc.) in 100 mL of sterilized water, adding 1 N·HCl and adjusting the pH to 7. Peptide 1 and peptide 9 were dissolved in the base to 0.1%. Each eye drops was stored at room temperature. Each eye drop was initially administered 3 weeks after administration of capsaicin, 3 times every day, in an amount of 10 μL for each eye.

[Measurement of lacrimal fluid volume]

The lacrimal fluid volume was measured by inserting ZoneQuick (Ayumi Pharmaceutical Corp.) inserted into the lower external eyelids of rats, and after 20 seconds, measuring the length of the red-stained portions using the included scale. The lacrimal fluid volume was measured 10 minutes and 5 minutes before eye dropping of the peptide, and the average value was recorded as the lacrimal fluid volume before eye dropping. The lacrimal fluid volume 5 minutes after eye dropping of the peptide was determined as the difference in lacrimal fluid volume compared to before eye dropping. The results are shown in Fig. 3.

In the rats that had developed neurotrophic keratitis, eye dropping of peptide 1 and peptide 9 produced similar lacrimal secretion effects.

[Observation of SPK]

SPK was evaluated 2 weeks after the start of eye dropping. Under isoflurane inhalation anesthesia, 1 μL of an aqueous solution of 1% fluorescein sodium dissolved in PBS (Wako Pure Chemical Industries, Ltd.) was dropped onto the eye surface, the eye was forcibly closed for 1 minute, and then the excess fluorescein staining solution was washed off with physiological saline. The eye surface was then observed with a slit lamp microscope, and the degree of corneal damage was evaluated by the method of Murakami et al., Atarashii Ganka 21 (1):87-90 (2004). Specifically, the cornea was divided into 3 regions, the top, center and bottom, and a score was assigned to each region on the following scale, with the value of the total of the scores of the 3 regions being recorded as the SPK score. <Scale> 0: No punctate stains, 1: Sparse (separated punctate fluorescein stains), 2: Intermediate (between 1 and 3), 3: Dense (almost all punctate fluorescein stains adjacent). The results are shown in Fig. 4.

Eye dropping of peptide 1 and peptide 9 improved the increased SPK score caused by capsaicin administration.

[Measurement of corneal sensation]

Corneal sensation is measured using a Cochet-Bonnet corneal sensory meter (Handaya Shoten). The filament length of the corneal sensory meter is set to 60 mm, and the filament is contacted vertically at the center of the cornea for stimulation. The eyelid response is observed, recording the filament length at which a response is obtained. When no response is seen, measurement is made while shortening the length by 5 mm at a time. This procedure is repeated 3 times, and the average value for the obtained filament lengths is recorded as the corneal sensation threshold (CST) for that individual.
Eye dropping of peptide 1 and peptide 9 improves reduction in corneal sensation caused by capsaicin administration.

[Corneal nerve staining]

After euthanizing rats, the eye bulbs are extracted and immersed in Zamboni solution (product of Wako Pure Chemical Industries, Ltd.), and allowed to stand for 15 minutes at room temperature. The corneas are harvested from the eye bulbs and reimmersed in Zamboni solution, and then allowed to stand for 45 minutes at room temperature. The solution is then replaced with 30% sucrose-containing 0.1 M phosphate buffer. After confirming deposition of corneal tissue, a slit is formed using scissors and the tissue is immersed in 0.1 M phosphate buffer (pH 5.3) containing 0.1% EDTA and 0.01% type IV-S hyaluronidase (Sigma) and allowed to stand overnight at 37°C. After washing with 0.3% TritonX-100-containing PBS-T, it is immersed in 1% BSA-containing PBS-T, for blocking at room temperature for 2 hours. Alexa Fluor 488-binding mouse anti-β-tubulin Class III antibody (BD Co.) is reacted with this overnight at room temperature. After washing with PBS-T, the corneal tissue is attached to slide glass and encapsulated in Vectashield hard set mounting medium. The encapsulated sample is observed with a confocal microscope and an image of the subcorneal plexus is taken. The nerve density of the swirl structure in the nerve layer is quantified by conducting Sholl analysis using image analysis software (Image J).
Eye dropping of peptide 1 and peptide 9 improves reduction in nerve density caused by capsaicin administration.

Example 10: Evaluation of axon elongation effect of PACAP27 and its stabilized peptide on rat trigeminal nerve cells

[Isolation and culturing of trigeminal nerve]

Rats (Slc: Sprague-Dawley, 17-day-old, including female and male) purchased from Japan SLC, Inc. were euthanized by carbon dioxide gas inhalation and the trigeminal ganglion was harvested. The harvested trigeminal ganglion was washed with Hanks' balanced salt solution (HBSS) and then treated with collagenase A (Roche) for 30 minutes at 37°C. A neuron dispersion (Wako) was then used according to protocol for dispersion of the cells. Debris removal solution (Miltenyi biotec) and Myelin removal beads II (Miltenyi biotec) were used according to protocol to remove the debris and myelin. After suspending the isolated trigeminal nerve cells in culture solution, they were seeded onto a polylysine/laminin-coated 8-well chamber slide (Corning) at $2.5 \times 10^3$ cells/well. The culture solution used was Neurobasal-A containing B27 supplement (final concentration: 2%) and GlutaMAX (final concentration: 2 mM) and penicillin/streptomycin (final concentration: 1%). The culturing conditions were a carbon dioxide concentration of 5%, an air concentration of 95%, a humidity of 100% and a temperature of 37°C. Seeding was followed by culturing for 24 hours, with replacement of culture solution containing peptide 1, peptide 9, peptide 12, peptide 18 or peptide 31 to a final concentration of 1 μM. The control was replaced with peptide-free culture solution. Culture medium replacement was followed by further culturing for 24 hours.

2. Staining

The culture medium was replaced with peptide-containing culture solution, and the rat trigeminal nerve cells cultured for 24 hours were immersed in a 2% paraformaldehyde solution at room temperature for 20 minutes for fixing. The cells were washed with PBS, and then 2% bovine serum albumin containing 0.1% TritonX-100 was added and reaction was conducted for 30 minutes. After washing with PBS, it was reacted with phospho-neurofilament H antibody (Merck, MAB1592-C) specifically recognizing neurofilaments forming the neuron bodies and neurites, for 1 hour at room temperature. After reaction for 1 hour, a fluorescent-labeled secondary antibody (Invitrogen Corp., #A-11031) was reacted at room temperature for 1 hour for fluorescent staining of the sample, and the stained cells were observed under a fluorescent microscope. The stained cell image was captured as an image from the fluorescent microscope and loaded into a computer.

3. Image analysis

In order to evaluate the extent of neurite formation by the rat trigeminal nerve cells, cells with neurites having lengths of at least twice the cell body diameter in the stained cell image in the computer were counted as neurite-forming cells, and the percentage (%) of this number of cells over the total cell count was calculated (Otori Y, Wei JY, Barnstable CJ. Invest. Ophthalmol Vis Sci (1998) 39, 972-981). The results are shown in Fig. 5 (N = 8). The significant difference with respect to the control was determined by Dunnett's test ($P < 0.001$).

Peptide 1, peptide 9, peptide 12, peptide 18 and peptide 31 exhibited axon elongation-promoting effects on trigeminal nerve cells.

Formulation Examples

A medicine containing a peptide of the invention as an active ingredient can be produced by the following formulation. The medicine of the present invention will now be further illustrated by formulation examples, with the understanding that the invention is not limited to the formulation examples.

1. Capsules

| | |
|---|---|
| (1) Peptide 5 | 40 mg |
| (2) Lactose | 70 mg |
| (3) Microcrystalline cellulose | 9 mg |
| (4) Magnesium stearate | 1 mg |
| 1 capsule: | 120 mg |

After mixing the total amounts of (1), (2) and (3) and 1/2 of (4), the mixture is granulated. The remaining amount of (4) is added and the entire mixture is encapsulated into gelatin capsules.

2. Tablets

| | |
|---|---|
| (1) Peptide 6 | 40 mg |
| (2) Lactose | 58 mg |
| (3) Corn starch | 18 mg |
| (4) Microcrystalline cellulose | 3.5 mg |
| (5) Magnesium stearate | 0.5 mg |
| 1 tablet | 120 mg |

After mixing the total amounts of (1), (2) and (3), 2/3 of (4) and 1/2 of (5), the mixture is granulated. The remaining amounts of (4) and (5) are added to the granules, which are then pressure molded into tablets.

3. Vitreous injection

In 1 ml:

| | |
|---|---|
| (1) Peptide 5 | 40 mg |
| (2) Refined sucrose | 50 mg |
| (3) Sodium chloride | 2.34 mg |
| (4) Polysorbate 80 | q.s. |
| (5) Disodium hydrogen phosphate | q.s |
| (6) Sodium dihydrogen phosphate | q.s. |

(continued)

| | |
|---|---|
| (7) Sterilized purified water | q.s. |

Components (1) to (6) are dissolved in the sterilized purified water (7) to prepare a vitreous injection.

4. Ophthalmic drug

In 100 mL:

| | |
|---|---|
| (1) Peptide 6 | 100 mg |
| (2) Tometamol | 300 mg |
| (3) Sodium chloride | 900 mg |
| (4) Benzalkonium chloride | q.s. |
| (5) Sterilized purified water | q.s. |

Components (1) to (4) are dissolved in the sterilized purified water (5) and the pH is adjusted to prepare eye drops.

## Claims

**1.** A peptide for use in a method of preventing or treating neurotrophic keratitis, consisting of a stabilized sequence of the sequence represented by:

HSDGIFTDSYSRYRKQMAVKKYLAAVLGKRYKQRVKNK (SEQ ID NO: 1) or
HSDGIFTDSYSRYRKQMAVKKYLAAVL (SEQ ID NO: 2),
(A) wherein the peptide consisting of the stabilized sequence has the sequence listed as SEQ ID NO: 1 or 2 with the carboxyl groups of the aspartic acid residues at position 3 and/or position 8 replaced with tetrazole, or its partially modified sequence wherein the partially modified sequence is a sequence wherein amino acids are added to the N-terminal end or C-terminal end, and the peptide consisting of the stabilized sequence has affinity for PAC1R, VPAC1R and/or VPACR2, or
(B) wherein the peptide consisting of the stabilized sequence is a peptide with the sequence represented by: H-$X_1$-D-G-1-F-T-D-$X_2$-Y-$X_3$-R-Y-R-$X_4$-$X_5$-$X_6$-A-$X_7$-$X_8$-$X_9$-Y-L-A-A-V-$X_{10}$ (SEQ ID NO: 3)

{where $X_1$ is a neutral amino acid, $X_2$ is a neutral amino acid, $X_3$ is a neutral amino acid, $X_4$ is a basic amino acid, $X_5$ is a neutral amino acid or egTz, $X_6$ is a non-polar amino acid, $X_7$ is a non-polar amino acid, $X_8$ is a basic amino acid, $X_9$ is a basic amino acid and $X_{10}$ is a neutral amino acid},
with the carboxyl groups of the aspartic acid residues at position 3 and/or position 8 replaced by tetrazole, or its modified sequence wherein the modified sequence is a sequence with an addition of one or more amino acids, and/or wherein one or two amino acids are deleted,
wherein the peptide has affinity for PAC1R, VPAC1R and/or VPACR2, and the modified sequence is the sequence listed as SEQ ID NO: 3 having a deletion or addition of one or more amino acids.

**2.** The peptide for use according to claim 1(B), wherein the neutral amino acids of $X_1$, $X_2$ and $X_3$ are alanine or serine, and/or

wherein the basic amino acids of $X_4$, $X_8$ and $X_9$ are lysine or arginine, and/or
wherein $X_5$ is glutamine, alanine or egTz, and/or
wherein $X_6$ is methionine, norleucine, alanine or leucine, and/or
wherein $X_7$ is valine or alanine, and/or
wherein $X_{10}$ is leucine or alanine.

**3.** The peptide for use according to any one of claims 1(B) or 2, wherein the carboxyl groups of the aspartic acids at position 3 and position 8 of SEQ ID NO: 3 are replaced by tetrazole.

**4.** The peptide for use according to any one of claims (1B) or 2 to 3, wherein the N-terminus of the peptide is acetylated or mesylated.

**5.** The peptide for use according to any one of claims (1B) or 2 to 4, wherein the N-terminus of the peptide is acetylated.

**6.** The peptide for use according to any one of claims (1B) or 2 to 5, wherein one sequence selected from the group consisting of the following:

GKRYKQRVKNK (SEQ ID NO: 37);
GKRYKQRVKN (SEQ ID NO: 38);
GKRYKQRVK (SEQ ID NO: 39);
GKRYKQRV (SEQ ID NO: 40);
GKRYKQR (SEQ ID NO: 41);
GKRYKQ (SEQ ID NO: 42);
GKRYK (SEQ ID NO: 43);
GKRY (SEQ ID NO: 44);
GKR;
GRR;
GK;
GR; and
G;
is added to the C-terminus of the peptide.

**7.** A peptide for in vivo use in treating trigeminal nerve damage or in regenerating the trigeminal nerve by promoting nerve axon elongation, consisting of a stabilized sequence of the sequence represented by:

HSDGIFTDSYSRYRKQMAVKKYLAAVLGKRYKQRVKNK (SEQ ID NO: 1) or
HSDGIFTDSYSRYRKQMAVKKYLAAVL (SEQ ID NO: 2),

wherein the carboxyl groups of aspartic acid are replaced with tetrazole.

**8.** A peptide consisting of a stabilized sequence of the sequence represented by:

HSDGIFTDSYSRYRKQMAVKKYLAAVLGKRYKQRVKNK (SEQ ID NO: 1) or
HSDGIFTDSYSRYRKQMAVKKYLAAVL (SEQ ID NO: 2),
wherein the carboxyl groups of aspartic acid are replaced with tetrazole,
for use in the treatment of neurotrophic keratitis or nerve injury or for use in nerve regeneration treatment, by promoting nerve axon elongation.

**9.** A method for promoting nerve axon elongation *in vitro,* comprising culturing neurons in culture medium modified by addition of a peptide consisting of the stabilized sequence represented by:

HSDGIFTDSYSRYRKQMAVKKYLAAVLGKRYKQRVKNK (SEQ ID NO: 1) or
HSDGIFTDSYSRYRKQMAVKKYLAAVL (SEQ ID NO: 2),

wherein the carboxyl groups of aspartic acid in said sequences are replaced with tetrazole.

## Patentansprüche

**1.** Peptid zur Verwendung in einem Verfahren zur Vorbeugung oder Behandlung von neurotropher Keratitis ("neurotrophic keratitis"), bestehend aus einer stabilisierten Sequenz von der Sequenz dargestellt durch:

HSDGIFTDSYSRYRKQMAVKKYLAAVLGKRYKQRVKNK (SEQ ID NO: 1) oder
HSDGIFTDSYSRYRKQMAVKKYLAAVL (SEQ ID NO: 2),
(A) wobei das Peptid, das aus der stabilisierten Sequenz besteht, die Sequenz hat, die als SEQ ID NO: 1 oder 2 aufgeführt ist, mit den Carboxylgruppen der Asparaginsäurereste an Position 3 und/oder Position 8 durch Tetrazol ersetzt, oder deren teilweise modifizierte Sequenz, wobei die teilweise modifizierte Sequenz eine Sequenz ist, wobei Aminosäuren an das N-terminale Ende oder das C-terminale Ende hinzugefügt sind, und das Peptid, das aus der stabilisierten Sequenz besteht, Affinität zu PAC1R, VPAC1R und/oder VPACR2 hat, oder
(B) wobei das Peptid, das aus der stabilisierten Sequenz besteht, ein Peptid ist mit der Sequenz dargestellt durch:

H-$X_1$-D-G-I-F-T-D-$X_2$-Y-$X_3$-R-Y-R-$X_4$-$X_5$-$X_6$-A-$X_7$-$X_8$-$X_9$-Y-L-A-A-V-$X_{10}$ (SEQ ID NO: 3)

{wobei $X_1$ eine neutrale Aminosäure ist, $X_2$ eine neutrale Aminosäure ist, $X_3$ eine neutrale Aminosäure ist, $X_4$ eine basische Aminosäure ist, $X_5$ eine neutrale Aminosäure oder egTz ist, $X_6$ eine unpolare Aminosäure ist, $X_7$ eine unpolare Aminosäure ist, $X_8$ eine basische Aminosäure ist, Xg eine basische Aminosäure ist und $X_{10}$ eine neutrale Aminosäure ist},
mit den Carboxylgruppen der Asparaginsäurereste an Position 3 und/oder Position 8 durch Tetrazol ersetzt, oder deren modifizierte Sequenz, wobei die modifizierte Sequenz eine Sequenz mit einer Hinzufügung einer oder mehrerer Aminosäuren ist und/oder wobei eine oder zwei Aminosäuren deletiert sind,
wobei das Peptid Affinität zu PAC1R, VPAC1R und/oder VPACR2 hat und die modifizierte Sequenz die als SEQ ID NO: 3 aufgeführte Sequenz ist, die eine Deletion oder Addition einer oder mehrerer Aminosäuren hat.

**2.** Peptid zur Verwendung gemäß Anspruch 1(B), wobei die neutralen Aminosäuren von $X_1$, $X_2$ und $X_3$ Alanin oder Serin sind, und/oder

wobei die basischen Aminosäuren von $X_4$, $X_8$ und $X_9$ Lysin oder Arginin sind, und/oder
wobei $X_5$ Glutamin, Alanin oder egTz ist, und/oder
wobei $X_6$ Methionin, Norleucin, Alanin oder Leucin ist, und/oder
wobei $X_7$ Valin oder Alanin ist, und/oder
wobei $X_{10}$ Leucin oder Alanin ist.

**3.** Peptid zur Verwendung gemäß irgendeinem der Ansprüche 1(B) oder 2, wobei die Carboxylgruppen der Asparaginsäuren an Position 3 und Position 8 von SEQ ID NO: 3 durch Tetrazol ersetzt sind.

**4.** Peptid zur Verwendung gemäß irgendeinem der Ansprüche (1B) oder 2 bis 3, wobei der N-Terminus des Peptids acetyliert oder mesyliert ist.

**5.** Peptid zur Verwendung gemäß irgendeinem der Ansprüche (1B) oder 2 bis 4, wobei der N-Terminus des Peptids acetyliert ist.

**6.** Peptid zur Verwendung gemäß irgendeinem der Ansprüche (1B) oder 2 bis 5, wobei eine Sequenz ausgewählt aus der Gruppe bestehend aus den folgenden:

GKRYKQRVKNK (SEQ ID NO: 37);
GKRYKQRVKN (SEQ ID NO: 38);
GKRYKQRVK (SEQ ID NO: 39);
GKRYKQRV (SEQ ID NO: 40);
GKRYKQR (SEQ ID NO: 41);
GKRYKQ (SEQ ID NO: 42);
GKRYK (SEQ ID NO: 43);
GKRY (SEQ ID NO: 44);
GKR;
GRR;
GK;
GR; und
G;
an den C-Terminus des Peptids angefügt ist.

**7.** Peptid zur in vivo Verwendung zum Behandeln von Schäden am Trigeminusnerv oder zum Regenerieren des Trigeminusnervs durch Fördern der Verlängerung von Nervenaxonen, bestehend aus einer stabilisierten Sequenz der Sequenz dargestellt durch:

HSDGIFTDSYSRYRKQMAVKKYLAAVLGKRYKQRVKNK (SEQ ID NO: 1) oder
HSDGIFTDSYSRYRKQMAVKKYLAAVL (SEQ ID NO: 2),

wobei die Carboxylgruppen der Asparaginsäure durch Tetrazol ersetzt sind.

8. Peptid, bestehend aus einer stabilisierten Sequenz der Sequenz dargestellt durch:

HSDGIFTDSYSRYRKQMAVKKYLAAVLGKRYKQRVKNK (SEQ ID NO: 1) oder
HSDGIFTDSYSRYRKQMAVKKYLAAVL (SEQ ID NO: 2),
wobei die Carboxylgruppen der Asparaginsäure durch Tetrazol ersetzt sind,
zur Verwendung bei der Behandlung von neurotropher Keratitis oder Nervenverletzungen oder zur Verwendung bei der Nervenregenerationsbehandlung durch Fördern der Verlängerung von Nervenaxonen.

9. Verfahren zum Fördern der Verlängerung von Nervenaxonen *in vitro,* umfassend Kultivieren von Neuronen in einem Kulturmedium, das durch Zugabe eines Peptids modifiziert ist, das aus der stabilisierten Sequenz besteht, dargestellt durch:

HSDGIFTDSYSRYRKQMAVKKYLAAVLGKRYKQRVKNK (SEQ ID NO: 1) oder
HSDGIFTDSYSRYRKQMAVKKYLAAVL (SEQ ID NO: 2),

wobei die Carboxylgruppen der Asparaginsäure in den genannten Sequenzen durch Tetrazol ersetzt sind.

## Revendications

1. Peptide pour son utilisation dans un procédé de prévention ou de traitement de la kératite neurotrophique, constitué d'une séquence stabilisée de la séquence représentée par :

HSDGIFTDSYSRYRKQMAVKKYLAAVLGKRYKQRVKNK (SEQ ID NO : 1) ou
HSDGIFTDSYSRYRKQMAVKKYLAAVL (SEQ ID NO : 2),
(A)dans lequel le peptide constitué de la séquence stabilisée a la séquence répertoriée comme SEQ ID NO : 1 ou 2 avec les groupes carboxyle des résidus d'acide aspartique en position 3 et/ou en position 8 remplacés par du tétrazole, ou
sa séquence partiellement modifiée dans lequel la séquence partiellement modifiée est une séquence dans lequel des acides aminés sont ajoutés à l'extrémité N-terminale ou à l'extrémité C-terminale, et le peptide constitué de la séquence stabilisée a une affinité pour PAC1R, VPAC1R et/ou VPACR2, ou
(B)dans lequel le peptide constitué de la séquence stabilisée est un peptide avec la séquence représentée par : H-$X_4$-D-G-I-F-T-D-$X_2$-Y-$X_3$-R-Y-R-$X_4$-$X_5$-$X_6$-A-$X_7$-$X_8$-$X_9$-Y-L-A-A-V-$X_{10}$ (SEQ ID NO : 3) {où $X_1$ est un acide aminé neutre, $X_2$ est un acide aminé neutre, $X_3$ est un acide aminé neutre, $X_4$ est un acide aminé basique, $X_5$ est un acide aminé neutre ou egTz, $X_6$ est un acide aminé non polaire, $X_7$ est un acide aminé non polaire, $X_8$ est un acide aminé basique, $X_9$ est un acide aminé basique et $X_{10}$ est un acide aminé neutre},

avec les groupes carboxyle des résidus d'acide aspartique en position 3 et/ou en position 8 remplacés par du tétrazole, ou sa séquence modifiée dans lequel la séquence modifiée est une séquence avec un ou plusieurs acides aminés ajoutés, et/ou dans lequel un ou deux acides aminés sont supprimés,
dans lequel le peptide a une affinité pour PAC1R, VPAC1R et/ou VPACR2, et la séquence modifiée est la séquence répertoriée comme SEQ ID NO : 3 ayant une délétion ou un ajout d'un ou plusieurs acides aminés.

2. Peptide pour son utilisation selon la revendication 1(B), dans lequel les acides aminés neutres de $X_1$, $X_2$ et $X_3$ sont l'alanine ou la sérine, et/ou

dans lequel les acides aminés basiques de $X_4$, $X_8$ et $X_9$ sont la lysine ou l'arginine, et/ou
dans lequel $X_5$ est la glutamine, l'alanine ou l'egTz, et/ou
dans lequel $X_6$ est la méthionine, la norleucine, l'alanine ou la leucine, et/ou
dans lequel $X_7$ est la valine ou l'alanine, et/ou
dans lequel $X_{10}$ est la leucine ou l'alanine.

3. Peptide pour son utilisation selon l'une quelconque des revendications 1(B) ou la revendication 2, dans lequel les groupes carboxyle des acides aspartiques en position 3 et en position 8 de SEQ ID NO : 3 sont remplacés par du tétrazole.

4. Peptide pour son utilisation selon l'une quelconque des revendications (1B) ou 2 à 3, dans lequel l'extrémité N-terminale du peptide est acétylée ou mésylée.

5. Peptide pour son utilisation selon l'une quelconque des revendications (1B) ou 2 à 4, dans lequel l'extrémité N-terminale du peptide est acétylée.

6. Peptide pour son utilisation selon l'une quelconque des revendications (1B) ou 2 à 5, dans lequel une séquence sélectionnée dans le groupe constitué par ce qui suit :

GKRYKQRVKNK (SEQ ID NO : 37) ;
GKRYKQRVKN (SEQ ID NO : 38) ;
GKRYKQRVK (SEQ ID NO : 39) ;
GKRYKQRV (SEQ ID NO : 40) ;
GKRYKQR (SEQ ID NO : 41) ;
GKRYKQ (SEQ ID NO : 42) ;
GKRYK (SEQ ID NO : 43) ;
GKRY (SEQ ID NO : 44) ;
GKR ;
GRR ;
GK ;
GR ; et
G ;
est ajoutée à l'extrémité C-terminale du peptide.

7. Peptide pour son utilisation in vivo dans le traitement des lésions du nerf trijumeau ou dans la régénération du nerf trijumeau en favorisant l'allongement de l'axone nerveux, constitué d'une séquence stabilisée de la séquence représentée par :

HSDGIFTDSYSRYRKQMAVKKYLAAVLGKRYKQRVKNK (SEQ ID NO : 1) ou
HSDGIFTDSYSRYRKQMAVKKYLAAVL (SEQ ID NO : 2),

dans lequel les groupes carboxyle d'acide aspartique sont remplacés par du tétrazole.

8. Peptide constitué d'une séquence stabilisée de la séquence représentée par :

HSDGIFTDSYSRYRKQMAVKKYLAAVLGKRYKQRVKNK (SEQ ID NO : 1)
ou HSDGIFTDSYSRYRKQMAVKKYLAAVL (SEQ ID NO : 2),

dans lequel les groupes carboxyle d'acide aspartique sont remplacés par du tétrazole, pour son utilisation dans le traitement de la kératite neurotrophique ou une lésion nerveuse ou pour son utilisation dans le traitement de la régénération nerveuse, en favorisant l'allongement de l'axone nerveux.

9. Procédé pour favoriser l'allongement de l'axone nerveux *in vitro,* comprenant la culture de neurones dans un milieu de culture modifié par l'ajout d'un peptide constitué de la séquence stabilisée représentée par :

HSDGIFTDSYSRYRKQMAVKKYLAAVLGKRYKQRVKNK (SEQ ID NO : 1) ou
HSDGIFTDSYSRYRKQMAVKKYLAAVL (SEQ ID NO : 2),

dans lequel les groupes carboxyle d'acide aspartique dans lesdites séquences sont remplacés par du tétrazole.

FIG. 1

(PAC1R)

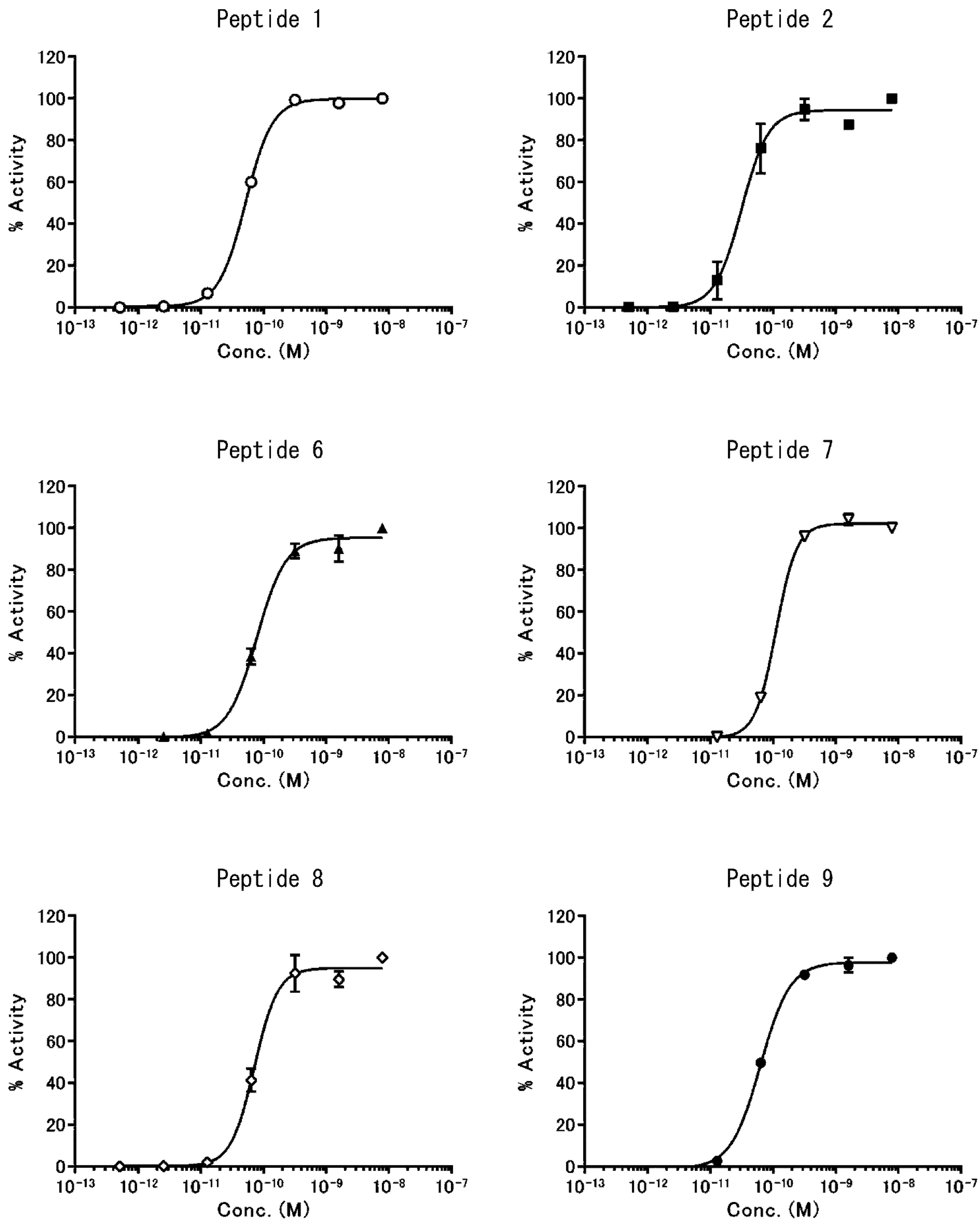
Peptide 1
Peptide 2
Peptide 6
Peptide 7
Peptide 8
Peptide 9
% Activity
0
20
40
60
80
100
120
$10^{-13}$
$10^{-12}$
$10^{-11}$
$10^{-10}$
$10^{-9}$
$10^{-8}$
$10^{-7}$
Conc. (M)

FIG. 2

(VPAC1R)

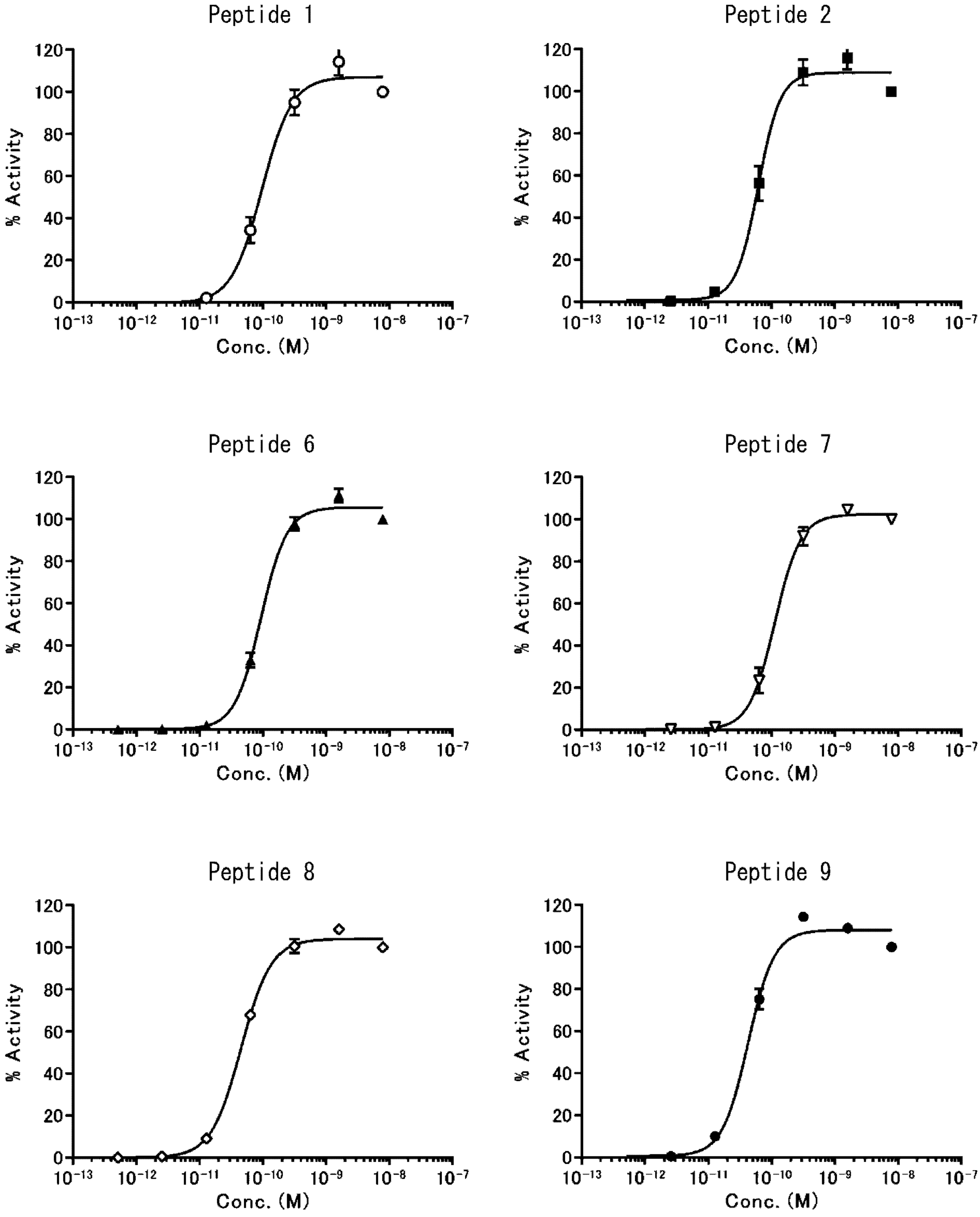
Peptide 1
Peptide 2
Peptide 6
Peptide 7
Peptide 8
Peptide 9
% Activity
Conc. (M)
0
20
40
60
80
100
120
$10^{-13}$
$10^{-12}$
$10^{-11}$
$10^{-10}$
$10^{-9}$
$10^{-8}$
$10^{-7}$

FIG. 3

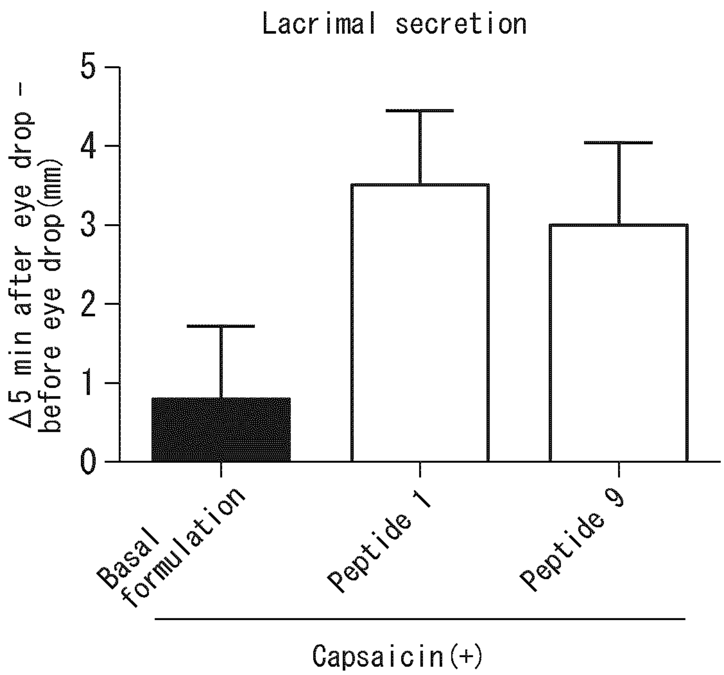
Lacrimal secretion
Δ5 min after eye drop - before eye drop (mm)
0
1
2
3
4
5
Basal formulation
Peptide 1
Peptide 9
Capsaicin(+)

FIG. 4

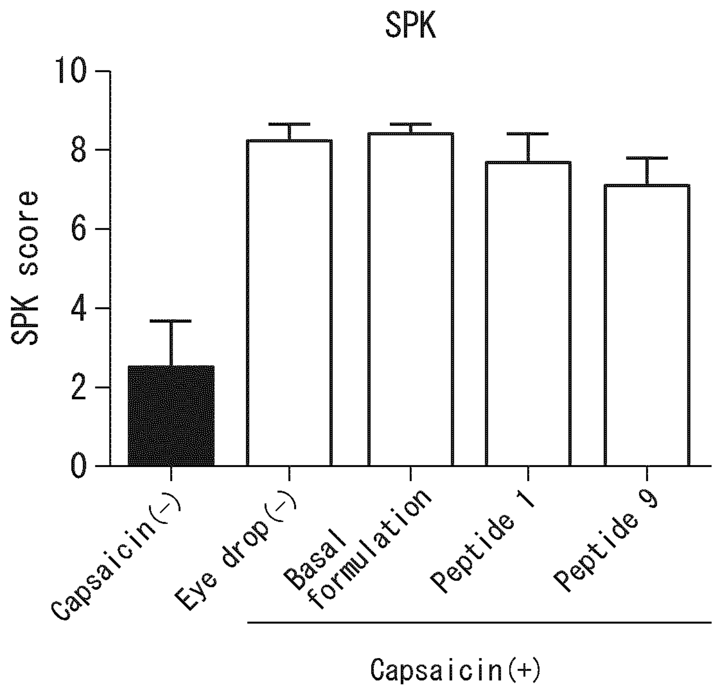
SPK
SPK score
0
2
4
6
8
10
Capsaicin(-)
Eye drop(-)
Basal formulation
Peptide 1
Peptide 9
Capsaicin(+)

FIG. 5

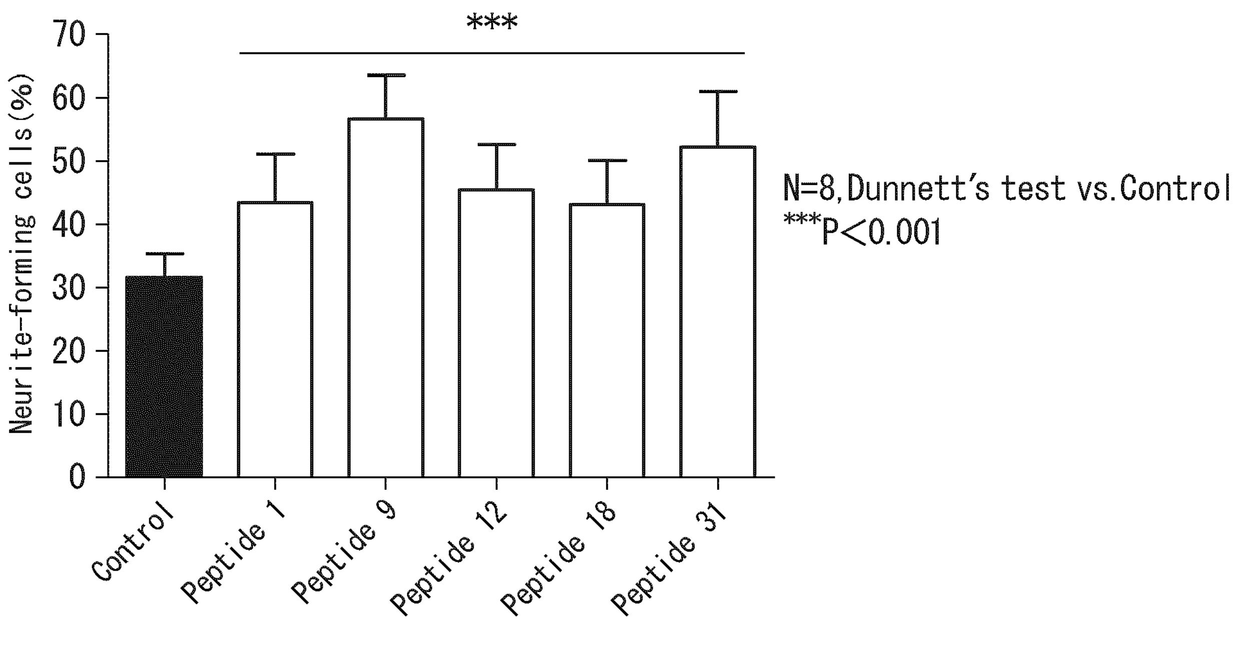
***
Neurite-forming cells(%)
70
60
50
40
30
20
10
0
Control
Peptide 1
Peptide 9
Peptide 12
Peptide 18
Peptide 31
N=8,Dunnett's test vs.Control
***P<0.001

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014510101 A
- JP 2012232952 A
- JP 2009269818 A
- WO 2005102375 A
- WO 2017027848 A


### Non-patent literature cited in the description


- **S. BOURGAULT**. *Current Medicinal Chemistry*, 2009, vol. 16, 4462-4480
- **LOUISE DICKSON**. *Pharmacology & Therapeutics*, 2009, vol. 12, 294-316
- **ALESSANDRO LAMBIASE**. *Investigative Ophthalmology & Visual Science*, 2012, vol. 53 (13), 8280-8287
- *Investigative Ophthalmology & Visual Science*, 2012, vol. 53 (13), 8280-8287
- **MURAKAMI et al.** *Atarashii Ganka*, 2004, vol. 21 (1), 87-90
- **OTORI Y** ; **WEI JY** ; **BARNSTABLE CJ**. *Invest. Ophthalmol Vis Sci*, 1998, vol. 39, 972-981